# EUROPEAN PATENT SPECIFICATION

(11) **EP 1 704 239 B1**
(45) Date of publication and mention of the grant of the patent: **14.01.2009**
(21) Application number: 05701883.0
(22) Date of filing: 14.01.2005
(51) Int. Cl.: C12N 15/867

(54) **INTEGRASE FUSION PROTEINS AND THEIR USE WITH INTEGRATING GENE THERAPY**
INTEGRASE-FUSIONSPROTEINE UND DEREN VERWENDUNG BEI DER INTEGRIERENDEN GENTHERAPIE
PROTEINES DE FUSION INTEGRASE ET LEUR UTILISATION EN THERAPIE GENIQUE D'INTEGRATION

(30) Priority: 14.01.2004 GB 0400814
(43) Date of publication of application: 27.09.2006
(73) Proprietor: Ark Therapeutics Ltd., London W1W 6XB (GB)
(72) Inventor: Ahlroth, Mervi A.I. Virtanen Institute, Fin-70211 Kuopio (FI); Schenkwein, Diana A.I. Virtanen Institute, Fin-70211 Kuopio (FI); Airenne, Kari A.I. Virtanen Institute, Fin-70211 Kuopio (FI); Yla-Herttuala, Seppo A.I. Virtanen Institute, Fin-70211 Kuopio (FI); Laitinen, Olli A.I. Virtanen Institute, Fin-70211 Kuopio (FI)
(74) Representative: Perry, Robert Edward
(86) International application number: PCT/GB2005/000115
(87) International publication number: WO 2005/068641

(56) References cited:
- WO-A-95/32225
- WO-A-96/35777
- HOLMES-SON MICHELLE L ET AL: "Integrase-LexA fusion proteins incorporated into human immunodeficiency virus type 1 that contains a catalytically inactive integrase gene are functional to mediate integration" JOURNAL OF VIROLOGY, vol. 74, no. 24, December 2000 (2000-12), pages 11548-11556, XP002331275 ISSN: 0022-538X
- BUSHMAN F D ET AL: "TETHERING HUMAN IMMUNODEFICIENCY VIRUS TYPE 1 PREINTEGRATION COMPLEXES TO TARGET DNA PROMOTES INTEGRATION AT NEARBY SITES" JOURNAL OF VIROLOGY, THE AMERICAN SOCIETY FOR MICROBIOLOGY, US, vol. 71, no. 1, January 1997 (1997-01), pages 458-464, XP002914950 ISSN: 0022-538X
- KATZ RICHARD A ET AL: "Targeting of retroviral integrase by fusion to a heterologous DNA binding domain: In vitro activities and incorporation of a fusion protein into viral particles" 1996, VIROLOGY, VOL. 217, NR. 1, PAGE(S) 178-190 , XP002331277 ISSN: 0042-6822 figure 1
- HOLMES-SON M L ET AL: "Molecular genetics and target site specificity of retroviral integration." ADVANCES IN GENETICS. 2001, vol. 43, 2001, pages 33-69, XP009048763 ISSN: 0065-2660
- MANNINO STEPHEN J ET AL: "Chemical mechanism of DNA cleavage by the homing endonuclease I-PpoI" BIOCHEMISTRY, vol. 38, no. 49, 7 December 1999 (1999-12-07), pages 16178-16186, XP002331276 ISSN: 0006-2960

## Description

### 1. FIELD OF THE INVENTION

This invention relates to integrase fusion proteins and their use with integrating gene therapy vectors.

### 2. BACKGROUND OF THE INVENTION

Integrating vectors and especially retrovirus-based vectors designed for gene therapy have gained much undesirable publicity because of the side-effects associated with retroviral gene therapy trials aimed at treating children suffering from the X-linked severe combined immunodeficiency disease (X-SCID). Although the treatment was clearly beneficial, two out of the ten treated children developed a leukaemia-like disease as a result of integration of the retroviral genome close to an oncogene (Marshall, 2003). These adverse effects have raised much concern about the safety of integrating vectors.

Hybrids vectors are capable of targeted transgene integration. Baculovirus-adeno-associated virus vectors (Palombo et al., 1998) targeted transgene integration into the AAVS1 site in approximately 41% of cases and Ad-AAV hybrids with efficiencies of 3-35% (Recchia et al., 1999). An AAV vector designed to mediate site-specific integration by a transient Rep expression reached targeting efficiencies of 42% (Satoh et al., 2000). There is a need for more efficient and safe gene therapy vectors, for targeted transgene integration.

The earliest *in vitro* assays studying the integration mechanisms of retroviruses used preintegration complexes (PICs) directly isolated from infected cells (Brown et al., 1987; Famet & Haseltine, 1990; Lee & Coffin, 1990). More recent assays use the recombinant purified IN (integrase) protein of different retroviruses in conjunction with short synthetic oligonucleotide substrates that mimic the ends of the viral DNA molecule. In optimal conditions, these assays have shown that the purified integrase alone can catalyse the major steps of integration, namely the 3' end processing and the 3' end joining (strand transfer) reactions (Bushman & Craigie, 1991; Craigie et al., 1990; Katz et al., 1990).

The 3' end processing activity of the IN requires the purified protein and radioactively labelled double stranded oligonucleotides containing sequences derived from the ends of either the U5 or the U3 LTRs (Chow, 1997; Brown, 1997). Gel electrophoresis is conventionally used to resolve the shortened products from the substrates. The 3' end-joining (strand transfer) reaction can be carried out with a similar composition, but the substrate is often "pre-processed", i.e. lacks the dinucleotides cleaved during the 3' modification step. The IN does not recognise special features of the integration target *in vitro,* so the target can either be the same molecule as the substrate or a circular plasmid DNA. The joining reaction can be assayed by the appearance of products that are longer in length than the input DNA in gel electrophoresis. The integration products may optionally be first phenol-chloroform extracted and ethanol precipitated, after which they can serve as templates for PCR (Chow, 1997). In addition to facilitating the detection of different sized products, PCR can also be used to facilitate the sequencing of the integration sites to reveal possible integration hot spots and the characteristic 5 bp duplications created by HIV-1 DNA integration. The site of the joining is largely random for the wt HIV-1 IN and the lengths of the products may therefore vary greatly (Chow, 1997; Brown, 1997).

The concerted integration (3' end joining or strand transfer) of two viral DNA ends into a target DNA is harder to achieve *in vitro.* Often the *in vitro* assays described above result in the joining of one DNA end into one strand of the target DNA (half-site integration) (Chow, 1997; Brown, 1997). The concerted integration can more accurately be studied using isolated PICs that (in addition to IN) provide the accessory enzymes required for full-site integration (see below). A circular plasmid DNA is used as the integration target and the event can be assayed by the presence of a selectable marker in the recombinant product or by southern blotting. This assay better resembles integration of retroviruses *in vivo,* as the recombinant HIV-1 IN has been shown to require the virally encoded nucleocapsid (NC) protein (Carteau et al., 1999) or the host-derived HMG-I(Y) (Hindmarsh et al., 1999) to perform efficient full-site integration *in vitro.* Even though the consensus is that at least the viral NC is required for full-site integration *in vitro* (e.g. Brown, 1997; Carteau et al., 1999), Sinha et al. (2002) suggested that using recombinant IN alone it is possible to obtain full-site integration of two donor termini without any cellular or viral protein cofactors *in vitro.* They concluded that the key factor allowing recombinant wild-type IN to mediate full-site integration is the avoidance of high IN concentrations in its purification and in the integration assay. However, the reaction end-products that they accounted for full-site integration products involved the concerted insertion of two LTR ends (U5 and U3 LTRs) per target DNA, not discriminating whether the LTRs were from the same substrate molecule or from two separate ones. Such concerted integration products are not comparable with the full-site integration products of retroviral genomes that form the provirus.

The HIV virion-associated proteins Vpr and Vpx can be used as vehicles to deliver proteins (viral or nonviral) into the virus particle by their expression in *trans* as heterologous fusion proteins (Wu et al., 1995, 1996a and b, 1997). Fusion of proteins to HIV-1 Vpr targets them to the newly formed virus in the virus producing cells via an interaction between Vpr and the C terminus of the p6 protein in Gag (Kondo & Gottlinger, 1996; Lu et al., 1995). It has thus been possible to achieve complementation of integrase (IN) function in IN-deleted HIV-1 virions (Wu et al., 1995) and to incorporate IN-fusion proteins (e.g. IN-LexA) into similar IN-defective virions (Holmes-Son & Chow, 2000). Also trans-packaged integrase fusion proteins can mediate correct integration and restore infectivity of the IN-defective viruses (Holmes-Son & Chow, 2002).

### 3 SUMMARY OF THE INVENTION

*In vitro* systems using viral preintegration complexes or purified IN (integrase protein) with short oligonucleotides have helped reveal important issues in the integration reaction as well as possible ways to inhibit retroviral infection in target cells. The HIV-1 integrase has been fused to sequence-specific DNA-binding proteins to test the possibility of directing retroviral integration. Testing the activity of the fusion proteins *in vitro* has showed that the sequence-specific proteins along with IN are capable of integrating substrate sequences at or close to the sites recognised by the proteins fused to IN.

A particular embodiment is a novel fusion protein consisting of HIV-1 IN and a sequence-specific homing endonuclease I*-Ppo*I (OR I*-Ppo*I's muntant from H98A) that may promote safe and targeted integration of gene therapy vectors. This may be useful because the homing endonuclease I*-Ppo*I recognises and cleaves its homing site present in the conserved 28S rDNA repeat of eukaryotes. While HIV-1 IN is designed to mediate the integration of retrovirus-like substrate sequences, I*-Ppo*I is designed to target the integration into the abundant rDNA locus in eukaryotic genomes.

A more general statement of the present invention is a method for the targeted integration of a transgene comprising retrovirus-like nucleic acid into a eukaryotic genome in vitro, in which the genome is targeted by a restriction enzyme that binds nucleic acid and the transgene is introduced at the binding site, wherein the endonuclease enzyme is specific to a site in an abundant rDNA locus and is fused to an integrase that mediates the introduction of the transgene.

The nature of the transgene is not critical; it is any that can provide a retrovirus-based vector for gene therapy. Such transgenes are known. The term "retrovirus-like" means any nucleic acid compatible with the desired integrase. For example the integrase is from the family of Retroviridae (e.g. murine retroviruses, lentiviruses such as HIV, SIV, FIN, EIAV, CAEV, BIV, VMV).

The genome of any eukaryote can in principle be modified according to the invention, although the method is especially suitable for humans. The intention is to direct integration, e.g. at a ribosomal site or another site in the genome of which there are many copies (i.e. redundancy) but no gene product. Where there are many copies of the target gene per genome, this has the advantage that the integration process may be more efficient (many targets), safe (because not all genomic ribosomal gene copies are needed) and reduced likelihood of interference with important genes, thereby reducing insertional mutagenesis. The endonuclease, e.g. I-PpoI, will cleave specifically at these multiple sites. Another suitable enzyme is CreI.

The integrase may be chosen according to the intended purpose. It will usually be a lentivirus integrase, of which HIV-1 integrase is an example.

The fusion protein, at least in specific embodiments thereof, and a polynucleotide encoding it, are novel. The protein may be produced by transforming a suitable host with an expression vector comprising the polynucleotide, and expressing the protein in or from the host. A preferred host is baculovirus.

The illustrative novel fusion protein IN-I*-Ppo*I is of use in gene therapy applications. The new fusion protein is capable of catalysing targeted integration of retrovirus-like DNAs into a benign locus in the human genome. The studies reported herein also included the preparation of DNA constructs required for testing the IN-I-*PpoI* fusion proteins' activity *in vitro.* The homing endonuclease I-*Ppo*I recognises and cleaves its target site present in the 28S rDNA of humans (Monnat et al., 1999). In conjunction with the HIV-1 IN, it is designed to target IN-mediated integration of the therapeutic genes into its rDNA site in humans. Heterologous gene expression has previously been reported from the I*-Ppo*I site in the yeast rDNA (Lin & Vogt, 2000); this shows that functional proteins can be expressed as pol I transcripts from the rDNA of eukaryotic cells.

### 4A DESCRIPTION OF THE INVENTION

The invention will be described by way of illustration with respect to a preferred embodiment. This involved producing the components required to test the integration activities and targeting abilities of a novel fusion protein consisting of HIV-1 IN and the HE I*-Ppo*I or I-PpoI's mutant form H98A. This aim included:
1- Creating the DNA constructs needed in expression of the IN-I*-Ppo*I and IN-H98A fusion proteins, as well as wt HIV-1 IN (control IN)
2- Designing and creating an LTR-flanked integration substrate
3- Generating an integration target plasmid containing the I-*Ppo*I recognition sequence
4- Production of the novel fusion proteins in bacterial hosts.

Some general aspects are: a polypeptide integrase, especially lentivirus integrase, and DNA-binding, especially with respect to human rDNA, activities; polynucleotides and vectors encoding it; its expression and production in a transformed host; compositions for administration comprising it; and its use in therapy, especially in targeted gene integration.

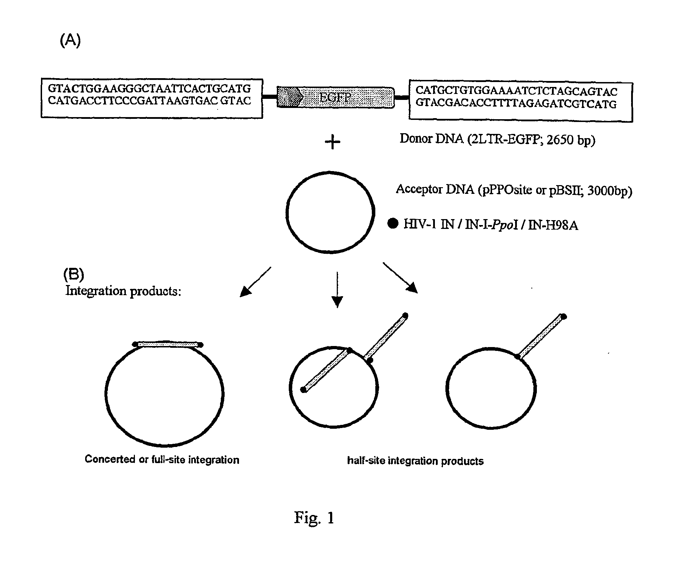

Being aware of the possible end results of *in vitro* integration assays, the work described herein was designed to provide the minimal components for the first step in testing the targeting abilities of the fusion protein IN-I-*Ppo*I. Possible half-site integration products can reveal that the HIV-1 integrase retains its activity in the context of the chimeric recombinant fusion protein. Analysis of the integration sites on the target DNA plasmid can shed light on the targeting possibilities by the proteins fused to HIV-1 integrase. Also, the endonucleolytic activity of I-*Ppo*I or its mutant form (I-*Ppo*I-) H98A fused to IN can be assessed by a simple cleavage experiment using the I*-Ppo*I recognitions site plasmid pPPOsite. In case the *in vitro* integration experiment should result in full-site integration products, a marker gene (EGFP) is included in the integration substrate to facilitate the screening of these events. Integration sites can be screened by PCR or RE digestion in either case, or radioactive labels can be incorporated to the integration substrate for faster observation by autoradiograms. An outline of the expected *in vitro* integration process is provided in Figure 1.

In Figure 1, (A) is a diagrammatic representation of a HIV-1 donor DNA substrate of ~2650 bps. The solid box represents an expression cassette for the EGFP (enhanced green fluorescent protein). (B) is a diagrammatic representation of an in vitro integration reaction with an acceptor DNA (pPPOsite and as a control pBluescript II), integration donor DNA (substrate from pB2LTR+EGFP) and purified HIV-1 IN (cIN), IN-I-*Ppo*I or IN-H98A proteins. Possible products include those that result from concerted integration of both donor DNA termini (left) and from non-concerted integration by two or more (middle) or one (right) donor DNA molecules via one-ended integration events.

To study the directed integration possibly achieved with the fusion proteins IN-I-*Ppo*I or IN-H98A, an integration target plasmid with the recognition site for I*-Ppo*I was created. The 15 bp sequence for the recognition site was adopted from previous studies using I*-Ppo*I (Mannino et al., 1999; Monnat et al., 1999; Ellison & Vogt, 1993; Argast et al., 1998). The recognition site was generated by hybridising two complementary oligonucleotides G515 and G517 (Appendix I, table I-4), after which the linker was cloned to the *Eco*RV site of pBluescript (Stratagene). Insertion of the linker was verified by both I*-Ppo*I (Promega) cleavage and by sequencing. pBluescript without the I*-Ppo*I recognition site was planned to serve as a control plasmid for the *in vitro* integration test to highlight the possible differences in the integration patterns of the wt HIV-1 IN (cIN in this study) and the proteins IN-I*-Ppo*I and IN-H98A .

The HIV-1 integrase, like other retroviral integrases, recognises special features at the ends of the viral DNA located in the U3 and U5 regions of the long terminal repeats (LTRs) (Brown, 1997). The LTR termini are the only viral sequences thought to be required in *cis* for recognition by the integration machinery of retroviruses. Short imperfect inverted repeats are present at the outer edges of the LTRs in both murine and avian retroviruses (reviewed by Reicin et al., 1995). Along with the subterminal CA located at the outermost positions 3 and 4 in retroviral DNA ends (positions 1 and 2 being the 3' end processed nucleotides, these sequences are both necessary and sufficient for correct proviral integration *in vitro* and *in vivo*. However, also sequences internal to the CA dinucleotide appear to be important for optimal IN activity (Brin & Leis, 2002a; Brin & Leis, 2002b; Brown, 1997). The terminal 15 bp of the HIV-1 LTRs have been shown to be crucial for correct 3' end processing and strand transfer reactions *in vitro* (Reicin et al., 1995; Brown, 1997). Longer substrates are used more efficiently than shorter ones by HIV-1 IN which indicates that binding interactions extend at least 14-21 bp inward from the viral DNA end. Brin and Leis (2002a) analysed the specific features of the HIV-1 LTRs and concluded that both the U3 and U5 LTR recognition sequences are required for IN-catalysed concerted DNA integration, even though the U5 LTRs are more efficient substrates for IN processing *in vitro* (Bushman & Craigie, 1991; Sherman et al., 1992). The positions 17-20 of the IN recognition sequences are needed for a concerted DNA integration mechanism, but the HIV-1 IN tolerates considerable variation in both the U3 and U5 termini extending from the invariant subterminal CA dinucleotide (Brin & Leis, 2002b).

For this study, the wild-type 20 bp HIV-1 IN recognition sites of the viral LTRs were adopted from Brin and Leis (2002a) who had used a mini donor DNA substrate for concerted integration *in vitro.* Deoxyoligonucleotides were designed in a way that the EGFP expression cassette could be cloned between the pBluescript contained 5' and 3' LTRs. In addition, the 5' and 3' LTRs were designed to contain a unique *Sca*I site that enables the digestion of the correctly blunt-ended LTR-EGFP-LTR (2LTR-EGFP) integration substrate construct. A blunt-ended integration substrate was created instead of a pre-processed one because it would allow detection of both the 3' end processing and the strand transfer reactions catalysed by IN. The EGFP marker gene was inserted between the LTRs to facilitate the recognition of full-site integration products that may be transformed into bacteria and selected in a biological assay (Brin & Leis, 2002a).

The construct pBVboostFG+EGFP, from which the EGFP expression cassette was cloned, bears promoters for protein expression in insect cells, mammalian cells and bacteria. All these promoters were retained in the LTR-EGFP construct cloned into pBluescript to facilitate the possible switch of the host for the future biological assay of *in vitro* integrants.

The integrase of HIV-1 has previously been shown to retain its activity when fused to DNA binding proteins and to become targeted by the sequence specific protein to which it is fused (Bushman, 1994; Bushman, 1995; Goulaouic & Chow, 1996; Pavletich & Pabo, 1991; Bushman & Miller, 1997; Holmes-Son & Chow, 2000). In the present study, the gene for the HIV-1 integrase (AF029884, clone HXB2) was fused to the homing endonuclease I-*Ppo*I (M38131) and to its mutant form H98A. Also a single form of the wt HIV-1 IN was subcloned to provide a control for the future *in vitro* tests. Fusion of the IN to its fusion partners was achieved through subcloning the cDNAs into pBluescript. Next, the cDNAs of IN*-*I*-Ppo*I, IN-H98A and cIN were transferred to pBVboostFG which is a universal expression vector compatible with recombinational cloning based on the bacteriophage λ recombination system (Landy, 1989) of the Gateway^{™} cloning technology (Invitrogen). The advantages of cloning the constructs IN-I-*Ppo*I, IN-H98A and cIN into this vector lie in the versatility of the vector driven expression of the cloned genes in various cells. Also, cloning the cDNAs into this vector is simple and has the advantage that baculoviral bacmids can easily be generated from the constructs residing in pBVboostFG. Baculoviruses are arthropod viruses that have long been used in expressing recombinant genes in insect cells and more recently as mammalian cell gene-delivery vectors (Kost & Condreay, 2002; Hüser & Hofmann, 2003).

As it had been possible to obtain catalytically active bacterially expressed HIV-1z IN in previous studies, IN and its fusion constructs IN-I-*Ppo*I and IN-H98A were first produced in *E. coli.* Two bacterial strains were used to find a suitable expression host. Expression of the control (wt) integrase and the fusion construct IN-H98A proteins in both the bacterial strains *E.coli* BL21 (DE3) and BL21-AI resulted in high protein expression levels of both recombinant proteins, as observed by specific anti-integrase and anti-polyhistidine antibody staining of immunoblots. The expression of the fusion protein IN*-*I*-Ppo*I was hard to achieve and only slight amounts of the protein could be detected in the immunoblots.

A possible explanation is that the fusion protein IN-I-*Ppo*I exhibits more sequence degeneracy than the wt I-*Ppo*I (perhaps due to unspecific DNA binding by IN), or has a novel enzymatic activity that is detrimental for bacteria. No growth inhibition was observed in insect cells that were tested for expression after experiments with bacteria. These results suggest that the novel fusion protein IN-I-*Ppo*I contains some enzymatic activity, perhaps enhanced DNA cleavage catalysis, and that the expression constructs themselves are functional. Furthermore, the good results obtained from expressing IN-I-*Ppo*I in insect cells overcome the problems associated with the production problems in bacteria.

Recombinant baculovirus genomes (from *Autographa californica* nuclear polyhedrosis virus; AcMNP) were created by transferring the cDNAs of cIN, IN*-*I*-Ppo*I and IN-H98A contained within the pBVboostFG to baculoviral genomes through a site-specific transposition mechanism (Landy, 1989; Bac-to-Bac Baculovirus Expression system, Invitrogen; Luckow et al., 1993). Protein production was then driven in insect cells and it proved to be successful. The purified proteins and an integration substrate may be introduced into the nuclei of different cells to observe the integration patterns in cellular DNA. The endonucleolytically active I-*Ppo*I may be toxic for eukaryotic cells, as less than 40% of human cells (HT-1080 human fibrosarcoma cells) have survived from constitutive I*-Ppo*I expression lasting for 15-18 days (Monnat et al., 1999). I*-Ppo*I expressed in human cells is capable of cleaving approximately 10% its homing sites present in the 28S rDNA after 24 and 48 hours after transfection of the HE encoding plasmid (Monnat et al., 1999). If its activity is too high also when fused to HIV-1 IN, the endonucleolytically inactive form H98A should provide a means to target integration into the rDNA without homing endonuclease catalysed cleavage of the site. Also, it should be possible to design an I*-Ppo*I enzyme with reduced DNA cleaving activity or modified sequence specificity since the structure of the enzyme and the active site residues are well known (Jurica & Stoddard, 1999; Galburt et al., 2000; Chevalier & Stoddard, 2001). A fusion protein that targets integration of substrate sequences resembling HIV-1 DNA may be incorporated into IN-mutated HIV-1 virions, for example using the in *trans* approach of Bushman and Miller (1997). Baculoviral hybrids may also be tested for the same approach.

In one embodiment of the invention, IN-fusion proteins are used in "their natural context", i.e. in association with the other HIV-1 proteins that are known to assist in the integration process. IN-I-*Ppo*I and its mutant form IN-H98A become targeted to the newly forming HIV-1 virus particles by the aid of the HIV-1 Vpr, to which IN-fusion proteins are fused. Vpr is cleaved from the fusion proteins by the viral protease after the new virus has budded. Targeted transgene integration can thus be tested by using the new fusion integrase containing lentiviruses in cell cultures and animals, or *in vitro* by extracting the IN-I-*Ppo*I/IN-H98A-containing preintegration complexes (PICs) and using these instead of the recombinantly produced proteins in the previously described *in vitro* integration assay.

In an example of this embodiment, the amino acid D64 (aspartate) in the IN's catalytic core domain was mutated to V (valine) via a megaprimer based method. This mutation is known to abolish enzymatic activities by IN. The mutated fragment has been cloned to the lentivirus production plasmid pMDLg/pRRE.

The IN-I-*Ppo*I and IN-H98A genes were cloned into an eukaryotic expression plasmid and the HIV-1 Vpr gene (from pLR2P; Beatrice Hahn, UAB) was cloned in front of the fusion genes. The Vpr gene is followed by a protease cleavage site to allow removal of Vpr from the IN-fusion proteins after trans-packaging.

The lentivirus vector was produced according to the standard protocol, except that the IN-H98A encoding expression plasmid was added to the transfected four-plasmid DNA mixture. In these preliminary trans-packaging tests, virus was produced using the wt IN encoding pMDLg/pRRE.

Trans-packaging of the Vpr-IN-H98A fusion proteins into new lentivirus vector particles was successful.

### 4b MATERIALS AND METHODS

### 4.1 General methods in DNA manipulation

### 4.1.1 PCR

PCR reactions were performed with a thermal cycler (PTC-200 Peltier Thermal Cycler, MJ Research) using the programs listed in Appendix II. Primers used in amplifying different templates are described in Appendix I. The genes for HIV-1 integrase, I-*Ppo*I and its mutant form I-*Ppo*Ih98a were amplified using *Pfu* -polymerase (MBI Fermentas). GW-PCR (subsection 4.3.1) was carried out using the FailSafe^{™} PCR PreMix selection Kit (Epicentre). In colony PCR (4.1.9) and other PCR reactions used to check different cloning constructs, DyNAzyme™ II DNA polymerase (Finnzymes) and its recommended buffer was used.

### 4.1.2 AGE and gel extraction

Throughout this study, gels for agarose gel electrophoresis (AGE) were prepared by dissolving 0.8 to 1 gram of agarose (Promega) in 100ml of TBE- or TAE-buffer (0.8 or 1 % gels, w/v). Ethidium bromide (EtBr) was added to a final concentration of 0.4 µg/ml before casting. Gels were run on 70-120 volts (V) for 1 to 4 hours and the DNA was visualised with an UV- Transilluminator (M-20 UVP, Upland, CA, USA).

For DNA gel extraction, a 0.8 % TBE- or TAE gel was cast. DNA bands to be extracted were excised from the gel using a scalpel. The DNA trapped in the slices was extracted using a gel extraction kit (GenElute™ Agarose Spin Columns, SIGMA) according to manufacturer's instructions.

### 4.1.3 DNA purification, concentration and precipitation

DNA from different origins (from gel extraction, PCR, digestions etc) was purified and concentrated using the Wizard® Plus DNA Clean Up Kit (Promega) or alternatively Na-Ac - alcohol- precipitation method (Sambrook & Russell, 2001) was used. DNA was usually eluted or dissolved in 30-50 µl of sterile endonuclease-free water. The concentration of DNA and its purity in the eluate were determined spectrophotometrically.

### 4.1.4 DNA concentration measurements

For DNA concentration measurements, samples were diluted in sterile water or TE (1:100). The concentration of DNA in the sample was spectrophotometrically determined by measuring the absorbance at 260 nm (and 280 nm), assuming that a solution of 50 ng/ml gives an A₂₆₀ value of 1. The purity of the sample was also determined from the ratio of the absorbance values at 260 and 280 nm (A₂₆₀/A₂₈₀) assuming that the ratio in a pure DNA solution is ~ 1.8 (Sambrook & Russell, 2001). Absorbance measurements were performed using an UV/Visible light spectrophotometre (Ultrospec 2000, Pharmacia Biotech).

### 4.1.5 DNA digestions

All digestions in this study were performed using restriction enzymes (REs) and their associated buffers from New England Biolabs (NEB) or MBI Fermentas. Digestions were carried out according to manufacturer's recommendations for the proper temperatures and buffer conditions for each enzyme. The DNA concentration in the digestion was adjusted to approximately 0.1 - 0.5 µg/µl. At least 1 U (unit) of enzyme was used for every µg of DNA, but less than 10% of the digestion volume consisted of the RE. Digestions were regularly carried out for 2 hours or over night (o/n).

When verifying plasmid preparations from different clonings by RE digestions (restriction analysis), the reaction was typically assembled as shown below, incubated for 1-2 hours at the appropriate temperature for each enzyme and run on an 1% TBE gel (w/v).

| | |
|---|---|
| 2 | µl plasmid DNA |
| 2 | µl 10 X RE buffer |
| 2 | µl 10 X BSA (for certain NEB enzymes only) |
| 0,5 | µl of each RE |
| H₂O ad 20µl | |

### 4.1.6 DNA ligations

Ligation of an insert into a linearised vector involves the formation of new phosphodiester bonds between adjacent 5'-phosphate and 3'-hydroxyl residues. In this study, ligation reactions were regularly performed using 5-15 U of the bacteriophage T4 DNA ligase (MBI), 1X T4 ligase buffer, variable amounts (50-150 ng) of digested vector and at least a 3 X molar excess of insert DNA over the vector DNA. All ligation components were mixed in a sterile Eppendorf tube and sterile endonuclease-free water was used to bring the reaction volume to 10 or 15 µl. Cohesive end ligations were usually incubated at room temperature (RT, 22°C) for 30-60 minutes and blunt end ligations at 16°C over night (o/n). Background ligation controls (water substituting for insert DNA) were always carried out along the insert-ligations in order to determine the background level of non-recombinants.

### 4.1.7 Bacterial transformation

Dilutions of the ligation mix (approximately 500 and 250 pg DNA/µl) were used to transform competent *E. coli* DH5α cells (Gibco) using 1 µl of each dilution per 30 µl of competent bacteria. Prior to transformation, T4 DNA-ligase was inactivated by incubating the ligation mixture at 65°C for 10 minutes. Transformation was carried out by the heat shock method as follows: the diluted ligation mix was mixed with DH5α cells in chilled eppendorf tubes, tapped gently and let stand on ice for 30 minutes. The bacteria were subjected to a heat shock at +42°C water bath for 40 seconds and placed on ice for 2 minutes. 1x SOC (80µl) was then added on the bacteria and the cells were let recover in a shaking incubator for one hour (37°C, 250 rpm). Cells were spread on LB (Luria-Bertani) -agar plates supplied with the appropriate antibiotic for selection of transformed bacteria. When using blue/white colour selection of recombinant pBluescript clones, 50µl of 2% X-Gal (5-bromo-4-chloro-3-indolyl-beta-D-galacoside) was spread on the surface of the plate and allowed to absorb for 30 to 60 minutes prior to plating the bacteria. 30 µl of IPTG (isopropyl thio-β-D-galactoside), an inducer for the *lacZ*-gene in pBluescript, was directly mixed with the bacteria upon plating.

In addition to the ligation control reaction, positive and negative transformation controls were carried out simultaneously and given all the same treatments as for the ligation mix reactions. A positive transformation control comprised bacteria transformed with intact plasmid DNA and a negative control transformation with sterile water instead of any DNA. A positive control shows that the transformation reaction procedure works efficiently and a negative control is used to reveal possible DNA contaminations during transformation. Agar plates were incubated at 37°C o/n.

### 4.1.8 Purification and analysis of plasmid DNA

The day following transformation, single white colonies were selected from LB plates and inoculated into 5 ml of 1 x LB supplemented with the appropriate antibiotic. The bacteria were grown in a shaking incubator (250 rpm at +37°C) for 12-16 hours to increase the plasmid yield. On the following day, 2-5 ml of each bacterial culture was harvested and the plasmid-DNA was isolated using a mini-scale plasmid preparation kit (Wizard® *Plus* SV Minipreps DNA Purification System, Promega), according to the manufacturer's protocol. Plasmid preparations were identified by different restriction enzyme digestions and agarose gel electrophoresis. Glycerol stocks were created from each succeeded clone by mixing equal volumes of bacterial o/n culture with 99+% glycerol (Sigma) and then stored at -20°C.

### 4.1.9 Colony PCR

Occasionally, cloning success was first verified by colony PCR instead of verification of mini-scale plasmid preparations. Colony-PCR allows rapid detection of cloning success using bacterial colonies as templates for PCR. For each set of colonies to be tested, a PCR premix (master mix) was prepared (see below). Premix was aliquot in sterile eppendorf tubes and the bacteria serving as template were introduced by gently touching the colony with the tip of a pipette and then flushing it in the PCR-tube, being careful not to pick the entire colony. Alternatively, a colony was suspended in 50µl of 1 X LB with the appropriate antibiotic, let grow for 2 hours in a shaking incubator (37°C, 250 rpm) and then 2µl of the culture was used for a modified colony- PCR reaction. With the latter procedure, numerous colonies could be screened simultaneously on a 96-well plate. PCR was carried out using the program DPESÄKE (Appendix II) and the reaction conditions listed below. The PCR-products were checked by AGE in a 1% TBE gel.

One 20 µl PCR-reaction was composed of:

| | |
|---|---|
| 15 | µl (or 13 µl) PCR-grade water, |
| 2 | µl 10X Dynazyme PCR buffer (with MgCl₂) |
| 1 | µl DMSO (Dimethylsulfoxide, final concentration 5%), |
| 0.5 | µl dNTPs (10 mM), |
| 0.5 | µl primer T3 |
| 0.5 | µl primer T7 (standard primers for pBluescript) |
| 0.5 | µl_(1 U) DyNAzyme™ II DNA polymerase (Finnzymes) |
| (2 | µl of bacterial culture or the picked colony as template) |

### 4.1.10 Creating DNA linkers by oligodeoxyribonucleotide hybridisation

Equal amounts of synthetic complementary oligodeoxyribonucleotides (1 nmol) were mixed in 100µl sterile endonuclease-free water or TE. The solution was incubated at 95°C for 10 min to denature the strands and allowed slowly to cool to room temperature (RT) inside the block. Slow cooling ensured the strands to anneal correctly without any uncomplementary pairing or unwanted secondary structure conformations.

### 4.1.11 Sequence verification of recombinant constructs

Sequencing of all DNA constructs was performed with two automated DNA Sequencers (ALF and ALFexpress, Pharmacia) in the DNA Synthesis and Sequencing Facility of the A.I Virtanen Institute for Molecular Sciences, Kuopio. One microgram of template DNA was required for each sequencing reaction. Primers used for sequencing different samples are listed in table 4.6-I. All sequencing results were analysed using DNAMAN (version 5.2.9, Lynnon BioSoft).

### 4.2 Creating expression constructs of IN-I-PpoI, IN-H98A and cIN

The genes for HIV-1 integrase (Groarke, Hughes, Dutko; Hong et al., 1993; Sioud & Drilca, 1991), I-*Ppo*I (Muscarella & Vogt 1989, Muscarella et al., 1990) and I-*Ppo*Ih98a were first PCR-amplified (Arnheim & Erlich 1992) and then subcloned into the EcoRV digested pBluescript. Subcloned genes were fused to create the constructs pIP (IN-I*-Ppo*I) and pIH (IN-H98A; see figure 4.5-1 for plasmid maps). These gene fusions were modified and transferred to an expression (Destination) vector pBVboostFG using GATEWAY™ Cloning Technology (GibcoBRL®, Life Technologies).

### 4.2.1 Subcloning the genes for IN, cIN, I-PpoI and H98A into pBluescript

The cDNAs for the HIV-1 IN, I*-Ppo*I and I-*Ppo*Ih98a genes were obtained from plasmids pLJS10, pCNPpo6 and pCNPpo6h98a, respectively. The plasmid pLJS10 was obtained through the AIDS Research and Reference Reagent Program, Division of AIDS, NIAID, NIH: pLJS10 from Drs. JM Groarke, JV Hughes, and FJ Dutko. Plasmids pCNPpo6 and pCNPpo6h98a were a kind gift from Prof. Raymond J. Monnat Jr., University of Washington, Seattle.

Specific oligonucleotide primers for each gene (Appendix I) were designed on the basis of sequence information obtained from the plasmids' providers and the GenBank (accession #s: IN - AF029884; I-PpoI - M38131; I-PpoI(h98a) - 1CYQ_C) All primers except 3'IN (synthesized in TAG Copenhagen A/S) were synthesized using an ABI DNA Synthesizer in the DNA Synthesis and Sequencing Facility of the A.I Virtanen Institute for Molecular Sciences, Kuopio. New restriction enzyme sites for cloning purposes were included in the primers (Appendix I).

PCR reactions were assembled as listed below and performed using the program DS2006 (Appendix II) for amplifying the genes IN, cIN, I-PpoI and H98A Primers F992 and 3'IN were used to amplify IN; F992 and 3'cIN were used for cIN. Both I-PpoI and H98A were amplified using the same primer set (F987 and G7), since the point mutation of the latter did not coincide with the primer sequences. All the inserts were amplified using recombinant *Pfu* polymerase (MBI Fermentas) in a 40 µl reaction composed of:

| | |
|---|---|
| 27 | µl PCR-grade water |
| 4 | µl 10X buffer for recombinant *Pfu* polymerase (+MgSO⁴) |
| 1 | µl DMSO (Dimethylsulfoxide, c_{final} 2,5%) |
| 1 | µl dNTPs (10 mM) |
| 2 | µl 5' primer (20µM) |
| 2 | µl 3' primer (20µM) |
| 1 | µl *Pfu* polymerase (2,5U) |
| 2 | µl template DNA (1:100 dilution of pLJS10, pCNPpo6 and pCNPpo6h98a) |

The PCR-reactions were checked by AGE running an aliquot of the reaction in a 1% TBE- gel. The remaining PCR product was then loaded on a 0.8% TAE- gel from which correct PCR-products were extracted. Bands corresponding to the expected PCR-products were gel extracted and purified as described under 4.1. The concentration of DNA and its purity in the eluate were determined spectrophotometrically (4.1.4).

PCR products amplified by *Pfu*-polymerase possess blunt ends, so blunt end cloning was used in subcloning the genes for IN, cIN, I-PpoI and H98A into the phagemid pBluescript (pBluescript® II SK-/+, Stratagene). pBluescript was linearised by digesting it with the blunt end cutter *Eco*RV (MBI). After 2 hours of incubation at +37° C, the digestion was checked by running an aliquot in a 1% TBE gel. To separate the linearised vector from the remaining amount of intact, undigested supercoiled DNA, the entire sample was gel extracted using GenElute™ Agarose Spin Columns and purified with the DNA Clean Up Kit.

Blunt-end ligation of IN, cIN, I-PpoI and H98A into the *EcoR*V digested pBluescript was performed by incubating each ligation mix (100ng of the vector, 2µl T4 ligase (10U), 1 µl 10 X T4 ligase buffer, 100 to 125 ng of insert DNA (gel extracted and purified PCR products) and 3,5 µl H₂O) at +16°C o/n.

The ligation mixtures were transformed into competent *E.coli* DH5α cells as described in subsection 4.1.7. Bacteria were plated on LB plates supplemented with ampicillin (75 µg/ml) and 50 µl X-gal. 30µl of IPTG was mixed with the bacteria prior to plating. Plates were incubated at 37°C o/n. The following day, white isolated colonies were selected from the LBₐₘₚ plates, cultured in 5ml LBₐₘₚ media and the culture was processed to plasmid preparations as described in section 4.1.8. Plasmid preparations were identified by restriction analysis and gel electrophoresis. The IN and cIN mini preps were digested with *EcoR*I (NEB). Correct preparations were further verified by digesting with *Pac*I and *Xba*I (NEB) (*Pac*I and *Sfi*I for cIN-clones), the sites of which were introduced into the IN-gene during PCR. Similarly, the plasmids containing I-PpoI or H98A were verified by a *Bam*HI-digestion, and a *Spe*I*-Sfi*I- double digestion (NEB). All digestions were checked by AGE as described earlier. Correct clones in pBluescript were named pBIN for integrase, pBcIN for control integrase, pBIPpoI for I-PpoI and pBH98A for H98A.

### 4.2.2 Creating fusion genes from IN and I-PpoI/H98A

The fusion of the two genes (IN with either I-PpoI or H98A) was achieved by restriction enzyme based subcloning (Sambrook & Russel, 2001). First, the orientations of the inserts in pBluescript were deduced by different RE-digestions. The IN gene was found to lie in pBluescript in an orientation, where it could be detached from the plasmid by a *Xba*I-digestion from its 3' end, but stay attached to the plasmid from its 5' end. I-PpoI and H98A were digested with *Spe*I and found to detach from their plasmids.

pBIN was digested with *Xba*I to create cohesive ends (staggered cut ends, that can be ligated together) for the *Spe*I digested pBIPpoI or pBH98A fragments to be inserted. 2 µl of SAP (Shrimp alkaline phosphatase, 1un/µl) was added in the pBIN-digestion to minimise the background level, i.e. to prevent unligated pBIN plasmid from self-ligating and transforming along the ligation mix. The I-PpoI and H98A inserts were digested from their plasmids using *Spe*I and the fragments were extracted from a 0.8% TBE gel. The purified I-PpoI and H98A fragments were individually ligated to the linearised, purified pBIN using the same conditions as described earlier (section 4.1.6). Ligation mixtures were transformed as described in section 4.1.7. Instead of extracting the plasmid DNA from the resulting colonies, successful clones were verified by colony PCR (4.1.9). Colonies giving the expected result were inoculated in 5ml of LBₐₘₚ and processed to mini preps as in section 4.1.8. Mini preps were verified by *Pvu*II digestions and AGE. The orientation of the two cDNAs with respect to each other was deduced from the digested samples. Correctly orientated clones were named pIP for integrase-I-PpoI fusion and pIH for integrase-H98A fusions. One clone of each construct was verified by sequencing (4.6).

### 4.2.3 Correction of mutations

Sequencing of the clones pIP5 and pIH5 revealed a nonsense mutation in position 212 of the IN gene that was absent in the pBcIN5 clone. pIP5 and pIH5 were repaired taking advantage of pBcIN5 as follows: a fragment of 527 bps was digested from pIP5 and pIH5 with *Pac*I and *Afl*II and replaced by the corresponding but unmutated fragment from pBcIN5. The plasmid backbone from pIP5 and pIH5 was gel extracted and the insert fragment containing the mutation was discarded. The correcting fragment cut from pBcIN was isolated and purified and then used for ligation into the digested pIP and pIH -plasmids. Ligations, transformations and plasmid isolations were carried out as described earlier under 4.1. A successful clone could only be verified by sequencing. Clones named pkorj.IP3, pkorj.IH7 were sequenced with the primers listed in table 4.6-I.

### 4.2.4 Construction of expression vectors

To convert the fusion genes IN-I-PpoI and IN-H98A, as well as the c-IN clone, into constructs that could be expressed in bacterial, insect and mammalian cells, all the inserts were subcloned into new expression vectors. Recombination-based GATEWAY™ Cloning Technology (GibcoBRL®, Life Technologies) was used first to introduce the GW-PCR-modified genes into the Donor plasmid pDONR™ (Invitrogen) via a recombination based BP-reaction. From this plasmid the genes were again transferred via a LR reaction to a modified Destination Vector pBVboostFG that has the promoter elements necessary for transgene expression in different organisms. Polyhistidine tags (6 X His) were introduced to the 5' ends of the fusion genes.

### 4.2.4.1 Gateway (GW)-PCR

To create Entry Clones of pkorj.IP3, pkorj.IH7 and pBcIN5 by the GATEWAY-recombination technology, DNA elements (*att*B1 and *att*B2) were introduced in the 5' end of the genes by PCR. Primers (Appendix I, table I-2) were designed to contain the sequences *att*B1 and *att*B2 and the 5' primer was further modified to contain six extra histidine encoding codons to aid in future protein purification. PCR was performed with the FailSafe^{™} PCR PreMix selection Kit (Epicentre) using the kit included buffers A to L (buffered salt solutions containing dNTPs, various amounts of MgCl₂ and FailSafe PCR Enhancer with betaine), FailSafe PCR Enzyme Mix (DNA polymerases) and the program GW-2701 or GW-cIN (Appendix II). 1-3 ng of each template (pkorj.IP3, pkorj.IH7 or pBcIN) was used for a 50 µl PCR reaction. The products were gel-extracted from a 0.8% TBE gel as described before and concentrated by ethanol precipitation.

### 4.2.4.2 Construction of final expression vectors via BP and LR reactions

BP reactions were carried out for the plasmids pkorj.IP3, pkorj.IH7 and pBcIN5 according to the manufacturer's protocol and the products were transformed into *E. coli* DH5α cells (4.1.7). Plasmid preparations were made as described in 4.1.8. The resulting Donor-plasmid preparations (Entry clones) were verified by *Pvu*II (MBI) digestions after which they served as templates for the subsequent reactions, the LR reactions. LR reactions, where the transgenes become transferred from the Donor vectors into the

Destination vectors (pBVboostFG in this study), were similarly carried out following the manufacturer's instructions and transformed into DH5α cells. Plasmid preparations from the resulting colonies were verified by *Pvu*II digestions. Gene constructs in the final Destination vectors were verified by sequencing using the primers G502, G550, G449 and G448 (Appendix I, Table I-3). Correct clones named pDIP2, pDIH1 and pDcIN1 were used for bacterial protein production (4.6).

### 4.3 Preparing the integration target plasmid (pPPOsite)

The sequence for the cleavage site of *I-PpoI* endonuclease was adopted from previous studies using the enzyme (Mannino et al., 1999; Monnat et al., 1999; Ellison & Vogt, 1993; Argast et al., 1998). To prepare a plasmid that contained a single specific cleavage site for I*-Ppo*I, a double-stranded oligonucleotide containing the I*-Ppo*I recognition sequence was inserted into the *EcoR*V site of pBluescript. Two 5' phosphorylated 15-mer complementary oligonucleotide strands G515 and G517 (Appendix I, table I-4) composing the recognition site were annealed to form a double stranded site (I-Ppo-oligo). Equal amounts of the strands were mixed and hybridised as described in section 4.1.10.

pBluescript was digested with *EcoR*V as described earlier and treated with SAP (2 U), gel extracted and purified as described in 4.1. The linker I-Ppo-oligo was ligated to the linearised and purified plasmid using a 15 X molar excess of the insert (17.5ng) over the digested plasmid (225 ng) and the reaction mixture was incubated at +16°C over the weekend (o/we). The diluted ligation mixture was transformed into DH5α cells with the heat shock method as described earlier. Mini scale plasmid preparations were processed from the cultures grown from selected bacterial colonies as in section 4.1.7 and verified by a digestion with I*-Ppo*I (Promega).

### 4.4 Creating a substrate for integration (pB2LTR+EGFP)

To generate an integration substrate for the *in vitro* study, a double stranded donor DNA resembling the viral long terminal repeat -ends was created. First, for both LTRs (3' and 5' LTR), two complementary and 5' phosphorylated 30-mer oligodeoxyribonucleotides were synthesised on the basis of sequence information obtained from studies using *in vitro* experiments with HIV-1 integrase (Brin and Leis, 2002a). Equimolar amounts (100 pmol) of the 5' and 3' LTR's (G604+G605 and G569+G570, respectively; Appendix I, table I-4) complementary oligonucleotides were annealed as before in 4.1.10.

pBluescript was digested with *Spe*I, extracted from a 0,8% TBE-gel and purified as described earlier. The linearised plasmid was then digested with *Kpn*I and treated with SAP (2 U), gel-extracted and purified. 200ng of the vector was used in a standard 15µl ligation reaction containing 2,5 µl of the 3' LTR preparation (25ng). Ligation was carried out for 30 minutes at room temperature, after which the volume was increased to 20µl by adding 0,5 µl T4 ligase buffer (10X), 1 µl T4 DNA ligase, 1 µl H₂O and 2,5 µl of the 5' LTR preparation (25ng). Ligation was continued for another 30 minutes, after which the ligase was inactivated and the mixture transformed into DH5α cells (4.1.7). During the following days, light blue colonies were processed to plasmid preparations, which were verified by a *Sca*I digestion. Before inserting the marker gene cassette between the LTRs, one of these pB2LTR-plasmid preparations (#1) was verified by sequencing.

The 2561 bp EGFP (enhanced green fluorescent) -cassette to be inserted between the LTR-sequences was *Sph*I digested from pBVboostFG+EGFP, gel-extracted and purified as described before. pB2LTR#1 (containing 5' and 3' LTRs) was also digested by *Sph*I and treated with SAP. The EGFP marker gene cassette was attached to the digested and purified pB1 in a 15 µl-ligation reaction containing 110 ng of the digested plasmid, three times its molar amount of the EGFP-insert (about 280 ng), 12,5 U T4 DNA ligase and 1,5 µl of 10X T4 DNA ligase buffer. Ligation was first carried out for 1 hour at RT and continued in a refrigerator (+8°C) o/we. Diluted ligation mixture and its controls were transformed into DH5α-*E. coli* cells. Ten resulting white colonies were selected for plasmid preparations which were checked by a ScaI-digestion. Correct clones were named pB2LTR+EGFP (numbers 1-10) and verified by a *Sca*I digestion and sequencing.

### 4.5 Sequence verifications of recombinant constructs

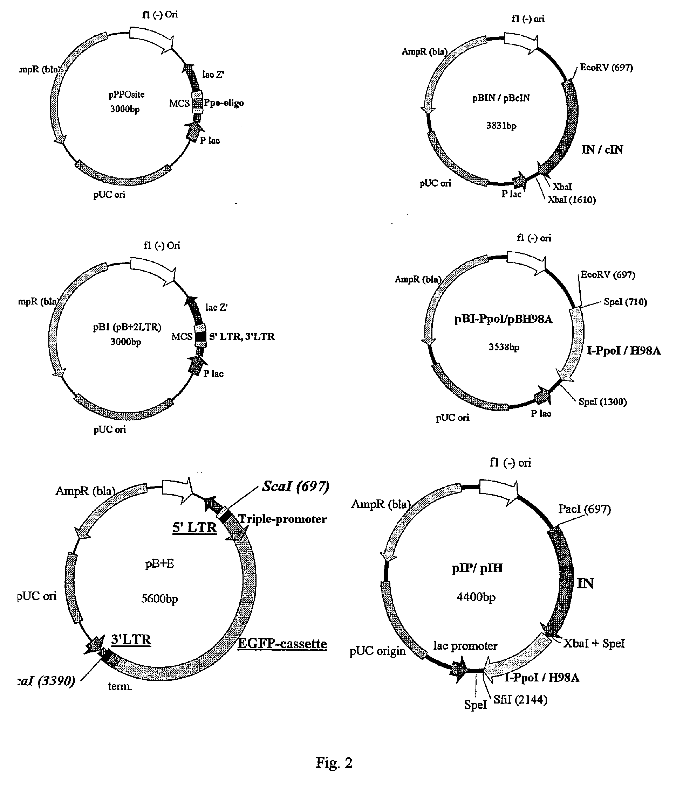

The primers used in sequencing different plasmids are listed in Appendix I. Plasmids pIP5 and pIH5 (from 4.2.3), as well as their corrected versions pkorj.IP3 and pkorj.IH7 (from 4.2.4) were sequenced with the four primers listed in table 4.5-I. pBcIN5, pPPOsite6 as well as the pB2LTR+E6 clone were sequenced with the primers T7 and T3. Expression clones pDIP2 and pDIH1 in pBVBoostFG were sequences with the primers G502, G550, G448 and G449. pDcIN was sequenced with the primers G502 and G550. All sequencing results were analysed using DNAMAN (version 5.2.9, Lynnon BioSoft). All the DNA constructs except the expression plasmids are illustrated in Figure 2.

**Table 4.5-I: Primers used for sequencing different DNA constructs. Sequences of the primers are listed in Appendix I.**

| sequencing primers/ templates | G448 | G449 | T3 | T7 | G502 | G550 |
|---|---|---|---|---|---|---|
| pIP5 | * | * | * | * | | |
| pIH5 | * | * | * | * | | |
| pBcIN5 | * | * | * | * | | |
| pkorj.IP2 | * | * | * | * | | |
| pkorj.IH7 | * | * | * | * | | |
| pDIP2 | * | * | | | * | * |
| pDIH1 | * | * | | | * | * |
| pDcIN1 | | | | | * | * |
| pPPOsite6 | | | * | * | | |
| pB2LTR1 | | | * | * | | |
| pB2LTR+E | | | * | * | | |
| 6 | | | | | | |

### 4.6 Bacterial protein expression

Competent *E.coli* strains BL21 (DE3) (Stratagene) and BL21-AI^{™} One Shot^{®} (Invitrogen life technologies, catalogue no. C6070-03) were used as expression hosts for protein production. Time points of 0h, 1h and 3h post-infection were taken during protein expression experiments and glycerol stocks were created from each tested clone. Protein production was analysed with SDS-PAGE (4.7.1) and Western Blotting (4.7.2).

### 4.6.1 Analytical scale bacterial protein production

Protein production was first carried out in the *E. coli* BL21 (DE3) strain. Bacteria were transformed with the expression vectors pDIP2, pDIH1 and pDcIN1 and pINSD.His. The His-tagged HIV-1 IN encoding plasmid pINSD.His was obtained through the AIDS Research and Reference Reagent Program, Division of AIDS, NIAID, NIH from Dr. Robert Craigie: pINSD.His. Transformation was performed as follows: The bacteria from a glycerol stock or an untransformed colony were spread on an LB plate the day before transformation in order to create a "fresh plate" and obtain viable colonies. The day following, isolated colonies were picked using a 10µl inoculation loop and suspended in 100µl of ice cold (+4 °C) CaCl₂. The suspended bacteria were incubated on ice for 15 minutes to render them competent for DNA uptake. DNA was added into the suspension (approximately 50-100ng, 0,5-1 µl), after which the mixture was again incubated on ice for 30 minutes. Next, the cells were subjected to a heat shock at 42° C for 45 seconds and immediately placed on ice for two minutes. 450 µl of S.O.C was added on the cells after which they were let recover in a shaking incubator (37° C, 230 rpm) for 30-60 minutes. Various amounts of the transformation mixtures (50-150 µl) were plated on LBg plates (LB plates supplied with gentamicin) and incubated at 37°C o/n.

The following day, a few colonies were selected from each transformed construct and cultured in 5ml of 1 X LB supplied with gentamicin (7µg/ml) at 37°C, 250 rpm, until the OD₆₀₀ (optical density of culture at 600nm) reached 0.6-1.0. From these initial cultures, 200µl were used to inoculate 3,8 ml fresh LBg (1:20 dilution of the initial culture). Untransformed BL21 (DE3) samples were processed as controls along the expression samples during all experiments.

The inoculated cultures were grown 1,5 to 3 hours at 37°C, 250 rpm, until they reached the mid-log phase (OD₆₀₀ ≈0,4). Protein production was induced by adding IPTG to a final concentration of 1 mM Before induction, a 1 ml sample (uninduced, 0h) was harvested from each culture. Additional 1 ml samples were collected 1h and 3 h (or every full hour until 4 h) post-induction. After harvesting, all samples were immediately pelleted by centrifugation (4000 x g, 2 min), suspended in 20-60µl sterile water and diluted in 4 X SDS-PAGE sample buffer. Samples were then heated to 95°C for 5 minutes to lyse the cells and stored at -20°C until analysed by SDS-PAGE. A part of the samples were not boiled at this point but stored immediately at -20°C.

Protein production was also tested in the *E. coli* strain BL21-AI^{™} One Shot^{®} (Invitrogen). One or half a vial (50 or 25 µl) of BL21-AI^{™} was used for each transformation of the constructs pDIP2, pDIH1 and pDcIN1. Transformation was carried out according to the basic transformation procedure described in section 4.1.7. Three transformants (colonies) were selected from each construct and initial culture was grown as described for BL21 (DE3). From these cultures, 200µl were used to inoculate 3,8 ml fresh LBg (1:20 dilution of the initial culture). Untransformed BL21-AI samples were processed as controls. The inoculated BL21-AI- cultures were grown 1,5 to 3 hours at 37°C or at 30°C, 250 rpm, until the OD₆₀₀ of the culture was approximately 0,4. Protein production was induced by adding L-arabinose (20% stock solution, Invitrogen) to a final concentration of 0.2% and IPTG to a final concentration of 1mM, 0.1 mM or 0.01 mM. Also, the impact of glucose in the growth medium was tested for production of the IN-I-PpoI protein (from pDIH). Glucose was added to a final concentration of 0.1% and all other steps were performed as previously. Expression cultures were usually grown at 37°C except when testing the impact of lower growth temperature (30°C) on protein degradation. Protein production samples were collected and processed as with BL21 (DE3) samples.

### 4.7 Characterisation of proteins

Samples from bacterial protein expression were prepared for SDS-PAGE immediately after harvesting. Before loading on the gel, samples were heated for 5 minutes at 95°C and placed on ice. The SDS-PAGE gels were blotted on nitrocellulose filters (Western blot) and the blotted proteins were identified by specific and sensitive antibody staining. Detection of the proteins with antibodies is based on alkaline phosphatase-activity conjugated to a secondary antibody (Blake et al., 1984).

### 4.7.1. SDS-PAGE

SDS-PAGE (Sodium dodecylsulfate-polyacrylamide gel electrophoresis) was performed using a Mini Protean II™ or a Mini Protean III™ device (BIO-RAD). First, a 10% running gel and a 4% stacking gel were cast (Appendix IV; Sambrook & Russell, 2001). 10µl of a molecular weight marker (SeeBlue ® Pre-Stained Standard, Invitrogen) and 20 to 50 µl of the samples and the controls were loaded in the wells of the stacking gel. The gel was first run with 100V for 10 to 20 minutes for the samples to migrate through the concentrating (stacking) gel. The volts were then raised to 180V (or 200V for Mini Protean III™ device) and the gel was run for additional ~60 minutes.

### 4.7.2 Immunoblotting (Western Blot)

The proteins resolved by SDS-PAGE were transferred to a nitrocellulose membrane (BIO-RAD Trans-Blot® Transfer Medium Pure Nitrocellulose Membrane, = 0.2 µm) using a BIO-RAD Mini Trans-Blot® device according to manufacturer's instructions. The transfer took place in cold Kodak buffer run with 100V for 1 hour, after which the blots were soaked in 0.5 M TBS buffer for 5 minutes. All proteins on the filter were visualised by staining the membranes with Ponceau S- dying solution (RT, 5 minutes, agitation) by covering the blots with the dye. The reaction was terminated by washing the membranes several times with distilled water. Membranes were then transferred to a blocking solution (5% non fat dried milk (NFDM) freshly made in TBS buffer) and agitated on a rotating shaker for 60 minutes at RT or o/n at 4°C.

Primary antibodies used in this study were (1) antisera to HIV-1 integrase peptide: aa 23-34 (NIH AIDS Research & Reference Reagent Program, catalogue #757) and (2) monoclonal Anti-poly-Histidine-Alkaline Phosphatase Conjugate from mouse (SIGMA). The secondary antibody for (1) was Goat Anti-Rabbit IgG (H+L) -AP Conjugate (BIO-RAD). All the incubations at the antibody detection phase were carried out on a rotating shaker.

After blocking, blots were rinsed with TBS-Tween for 5 minutes and incubated in the primary antibody -solution (1:2000 both anti-IN and anti-Histag in 5% NFDM freshly made in TBS tween, 10 ml / membrane) for 1 hour at RT. When using anti-integrase staining, unbound primary antibody was washed with TBS-Tween (4 X 5 minutes). The blots were then incubated in the enzyme-linked secondary antibody solution (goat anti-Rabbit IgG 1:3000 in 5% NFDM-TBS-Tween) for one hour at RT. Membranes were again rinsed with TBS-Tween (4 X 5 minutes) to eliminate unbound secondary antibody. When using anti-poly histidine antibody staining, blots were rinsed with TBS-Tween (4 X 5 minutes) after the first antibody probing and no secondary antibody was used.

Before colorimetric detection of proteins, membranes were equilibrated in APA-buffer by an incubation lasting approximately 5 minutes. Detection was achieved by incubating the filters in a NBT/BCIP substrate solution (Roche Diagnostics GmbH, Mannheim, Germany), 16µl/ml of NBT/BCIP in APA buffer, 5ml per filter at RT for 5 - 15 minutes. The colour reaction was terminated by washing the membranes several times with deionised water. Filters were dried and the results of the staining were analysed.

### 5 RESULTS

### 5.1 Creating expression constructs of cIN, IN-I-PpoI and IN-H98A

The plasmid pLJS10 contains the gene for the HIV-1 integrase (IN) (Hong et al., 1993; Sioud & Drilca, 1991). Plasmid pCNPpo6 contains the gene for a eukaryotic intron encoded homing endonuclease I*-Ppo*I from the myxomycete *Physarum Polycephalum* (Muscarella & Vogt, 1989; Muscarella et al., 1990). The *I-PpoI* gene (I-PpoI) in the plasmid pCNPpo6h98a has a mutation that replaces the amino acid residue histidine in position 98 into alanine. Histidine98 (H98) in the enzyme's active site is critical for efficient catalysis of DNA cleavage and its mutation into alanine severely diminishes the endonuclease activity of the protein (Mannino et al., 1999). Therefore, throughout this work the H98A mutated and endonucleolytically inactive I-PpoI gene is called H98A. Plasmids pLJS10, pCNPpo6 and pCNPpo6h98a were taken for use without preliminary verification of their sequences.

### 5.1.1 Subcloning the genes for IN, I-PpoI, H98A and cIN

The genes for HIV-1 integrase, I-PpoI and H98A were PCR-amplified as described in section 4.2.1. The primers (Appendix I, table I-1) used for PCR amplification were designed in a way to introduce new RE sites in the 5' and 3' ends of the genes. Two versions were created from the IN gene: one to be attached from its PCR-created 3' *Xba*I-end to 5'-*Spe*I ended I-PpoI and H98A (IN) and another to become expressed without fusion partners (cIN; control IN). The PCR reactions were checked on a 1 % TBE gel. The PCR products of the integrase gene were 870 bps long and the products from the I-PpoI and H98A genes were 570 bps.

In order to subclone the PCR products into pBluescript (Stratagene) the plasmid was digested with the blunt end cutter *Eco*RV, gel-extracted and purified. Gel extraction of the digested vector usually strongly reduces the background level resulting from non-recombinant parental vector. SAP was not used because the blunt-ended PCR products created by *Pfu* polymerase lack 5' phosphates needed in ligating the inserts into the linearised plasmid. The linearised pBluescript for gel extraction was 2961 bps long.

The PCR-amplified inserts IN, cIN, I-PpoI and H98A were ligated to the *Eco*RV digested pBluescript as described in subsection 4.2.2. The choice of the ligase was the bacteriophage T4 DNA ligase (MBI) owing its ability to join both blunt-ended and cohesive DNA fragments (Sambrook and Russel, 2001). Ligation mixtures of IN, cIN, I-PpoI and H98A with the *Eco*RV-digested pBluescript were transformed into *E. coli* DH5α cells. Prior to transformation, ligation mixtures were heated to 65°C for 10 minutes to inactivate the ligase. The heat-inactivation step can increase the number of transformants by two orders of magnitude (Michelsen, 1995). Of the colonies developed, mini preps of all the constructs were verified by restriction analysis (4.1.5) and AGE as described earlier.

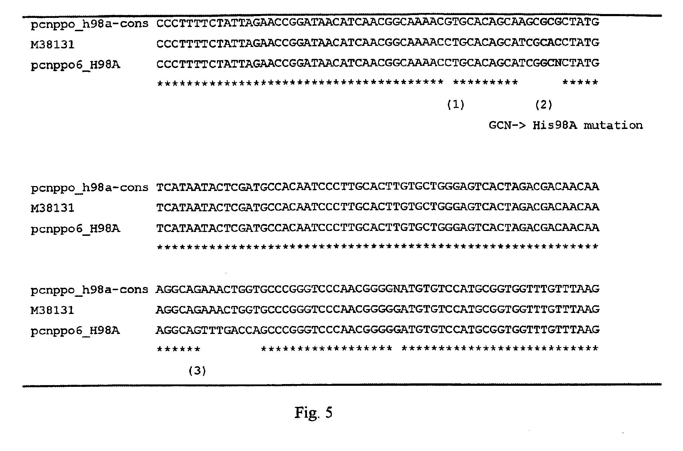

To screen the IN and cIN containing clones, mini preps were first digested with *Eco*RI*.* A successful clone was expected to appear as two bands of the sizes 465 and 3366 bps when visualised in a TBE-gel (Figure 5.1.1-III, lanes 3-5). Mini preps giving the expected result were further verified by a double digestion using REs *Pac*I and *Xba*I or *Pac*I and *Sfi*I for cIN-clones. These sites were introduced to the insert ends in the PCR reaction. The correct clones could be visualised by the detachment of an 870 bp sized fragment corresponding to the IN and cIN genes.

Similarly, the plasmids containing either I-PpoI or H98A were checked by digesting the plasmid preparations with *Bam*HI*.* A correct clone was expected to appear as two bands of 336 and 3202 bps. Clones were further verified by a double digestion with the insert specific restriction enzymes *Spe*I and *Sfi*I, which produced the 570 bp sized I-PpoI or H98A insert. Correct clones of the inserts in pBluescript were named pBIN for integrase, pBcIN for control integrase, pBIPpoI for I-PpoI, and pBH98A for H98A. One of each correct clone was chosen to be used in creating the fusion gene constructs.

### 5.1.2 Creating the fusion genes IN-I-PpoI and IN-H98A

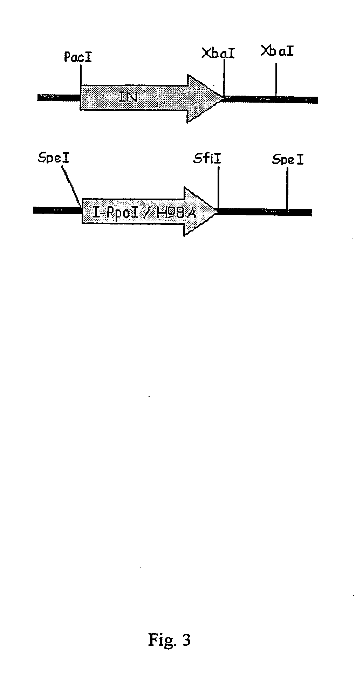

The IN gene residing in pBIN was fused to the I-PpoI and H98A fragments derived from pBIPpoI and pBH98A The orientations of the genes in their relevant plasmids were deduced by RE digestions. pBIN was digested with *Xba*I and its 5' end was found to stay attached to the plasmid while the gene's 3' end was freed along plasmid linearisation. This orientation was optimal, considering the fusion of the I-PpoI and H98A fragments into the integrase' 3' end. On the contrary, pBIPpoI and pBH98A clones were found to detach the inserted transgene when digested with *Spe*I, the enzyme producing compatible ends to the staggered cut created by *Xba*I. The detachment occurred because of another *Spe*I-site residing in the plasmid backbone, next to the inserts 3' end (Figure 3). Fusions of I-PpoI and H98A with IN were thus achieved by ligating the *SpeI* cut "fusion partner gene" fragment into the *Xba*I linearised pBIN.

Ligation mixtures containing the *Xba*I linearised pBIN and the *SpeI* digested fragments of I-PpoI or H98A were transformed into DH5α cells as described in 4.1.7. Clones with the wanted gene fusion were first screened by colony PCR (4.1.9) instead of directly proceeding to mini scale plasmid preparation from randomly selected colonies. A PCR product of ≈1610 bps (170 bp from pBluescript + 1440 from fused inserts) was expected from colonies having the IN gene fused to I-PpoI or H98A, whereas plasmids having only the IN gene inserted (the "background" clones) would give a product of ≈1040 bps (170+870 bp). Colonies giving the expected 1610 bp result were used to inoculate 5 ml LBₐₘₚ and the cultures were processed to mini preps as described before in 4.1.

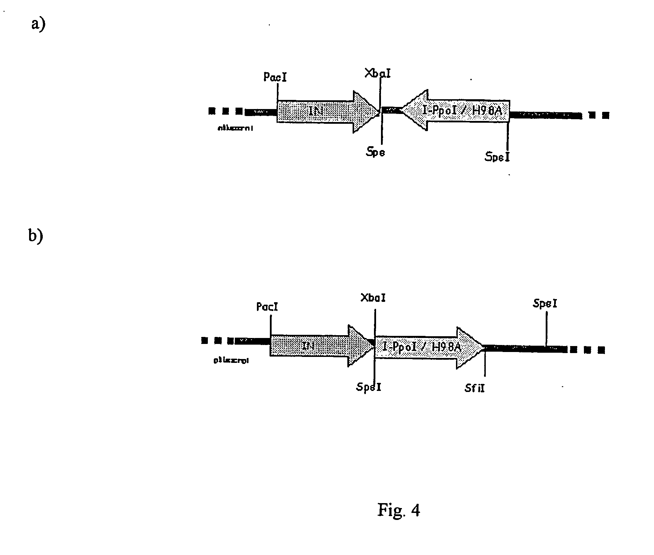

*Spe*I digested I-PpoI or H98A fragments could become ligated to the *Xba*I digested pBIN in two orientations (Figures 4a and 4b) because both 5' and 3' ends of the fragments had compatible ends with the digested vector. Plasmid preparations containing the IN-I-PpoI or IN-H98A fusions were verified by *Pvu*II digestions. In addition to showing the presence of the gene fusion, *Pvu*II digestion also revealed the orientation of the inserts in respect to each other. Correct orientation of the genes fused to IN was essential considering the functionality of the novel proteins encoded by the fusion gene.

The desired fusion (thus I-PpoI's or H98A's 5' end ligated to the integrase' 3' end, Figure 4b would result in four bands of the sizes 306, 728, 861 and about 2500 bps when digested with *Pvu*II. The wrong orientation would yield bands of 306, 403 and 1186 and approximately 2500 bps. The correctly orientated clones were named pIP for integrase-I-PpoI fusion and pIH for integrase-H98A fusions.

### 5.1.2.1 Sequencing results

Plasmids pBcIN5, pIP5 and pIH5 were sequenced with the pBluescript's standard primers T7 and T3. Each sequencing reaction gave a two-way sequence that spun about 500 bp of the transgenes' sequence in pBluescript. Plasmids pIP5 and pIH5 were additionally sequenced with the primers G448 and G449 (table 4.6-I). These primers were designed to anneal in opposite directions of the fusion gene's central region and they were used to amplify the sequence area not obtained using T3 and T7. Sequencing results were analysed using DNAMAN (version 5.2.9, Lynnon BioSoft) by comparing the obtained sequences to the template sequences (AF029884 (HIV-1 IN), M38131 (I-PpoI) and sequences of the plasmids pCNPpo6 and pCNPpo6_h98a.

The sequence of the control integrase gene in pBcIN5 was found to be correct apart from a silent point mutation that did not affect the amino acid residue encoded by the mutated codon. Plasmids pIP5 and pIH5, on the contrary, were both affected by a nonsense mutation in position 212 of the IN gene. The mutation would cause the expressed protein to become severely truncated, which made correction of the mutated plasmids essential. Also the I-PpoI and H98A encoding parts in plasmids pIP5 and pIH5 differed from the sequence information obtained from the plasmid's (pCNPPo6 and pCNPpo6h98a) provider. To verify this result, second clones from the fusion constructs (pIP2 and pIH7) were sequenced with the same primers as pIP5 and pIH5 (table 4.5-I).

The parental plasmids pCNPPo6 and pCNPpo6h98a were sequenced with the primers G7 and F987 (Appendix I). The actual sequence of these plasmids was found to differ from the initial sequence information given by the plasmids' provider and from each other in more than the expected one position (His98-mutation in pCNPpo6h98a). The sequences obtained from sequencing pCNPPo6 and pCNPpo6h98a were more similar to the wild type sequence of I-PpoI (M38131) than to the sequence information given by the plasmids' provider Figure 5). Also, the sequence differences outside the expected H98A mutation were found not to have effects on the amino acids encoded by the differing codons. Thus, also the I-PpoI and H98A encoding sequences obtained from pIP5 and pIH5 were correct as they corresponded to the wild type protein's amino acid sequence.

In Figure 5, represent identical sequence, gaps in the consensus ("star"-) row indicate differences in sequence. The base difference in the first gap (1) has no effect on amino acid encoded by the affected codon. Differences in (2) are also silent apart from the His98 mutation presented as GCN in the sequence obtained from pCNPpo6h98a's provider (codon showed in bolded, wt encodes for His and mutated codon for Ala). Case (3) indicates a sequence difference of the parental plasmid compared to wt sequence of I-PpoI, but the actual sequence of the plasmid pCNPpo6h98a was found to be identical to the wt sequence. The plasmid pCNPpo6 only differed from the wt sequence in the expected His98 encoding triplet.

### 5.1.3 Correction of mutations in pIP5 and pIH5

The IN gene in pBcIN5 did not contain the nonsense mutation found in position 212 of the pIP5 and pIH5 fusion insert plasmids. This was partly due to the separate PCR-reactions used to amplify integrase genes for different purposes (having different primers; Appendix I) and because of the possible heterogeneity of PCR products in one reaction.

The pIP5 and pIH5 plasmids harboring the nonsense mutation were repaired taking advantage of the unmutated IN gene in pBcIN. A fragment of 527 bps was detached from the unmutated pBcIN5 with PacI and AflII. pIP5 and pIH5 were similarly digested with the two enzymes and treated with SAP. The gel extracted and purified pBcIN fragment (527 bp) was ligated to the digested, gel extracted and purified plasmid backbones of pIP5 and pIH5 (3880 bp) lacking the corresponding fragment. A successful clone could only be verified by sequencing plasmid preparations. To increase the probability of finding a successful clone, mini preps were only prepared from transformants having low levels of background (ligation) control colonies. First sequenced samples of corrected pIP5 and pIH5 (plasmids pkorj.IP3, pkorj.IH7) were found to lack the nonsense mutation and correction of the mutation had succeeded. The new fusion gene preparations were used in Gateway PCR.

### 5.1.4 Gateway (GW) PCR

Gateway PCR (4.2.5.1) was performed using the *att*B1/B2-sites and a 5' Histag introducing primers (Appendix I, table I-2) and the FailSafe^{™} PCR PreMix selection Kit (Epicentre). The lengths of the primers G445 (5'), G238 (3' IN-Ppo and IN-H98A) and G402 (3' cIN) were 86, 70 and 54 bps, respectively, so 110 or 104 bps were newly introduced to the insert ends altogether. The expected size of the PCR products from amplifying pkorj.IP3, pkorj.IH7 was thus 1440 + 110 = 1550 bps and for pBcIN5 974 bps (870 + 104). PCR reactions were run on a 0,8% TBE gel and the correct sized bands were gel extracted and purified.

### 5.1.5 Construction of final expression vectors

BP reactions were carried out for the GW-PCR products of the plasmids pkorj.IP3, pkorj.IH7 and pBcIN5 following the manufacturer's protocol (GATEWAY^{™} Cloning Technology, GibcoBRL®, Life Technologies). The resulting Entry clones (pDONR™ plasmid preparations) were verified by *Pvu*II (MBI) digestions. Correct Entry clones were identified by the presence of three bands of 2480, 810 and 560 bps in the case of plasmid preparations created from the IN-Ppo and IN-H98A fusion genes. The Entry clones bearing the cIN gene (pEntryCIN 1-3) were similarly restriction analysed using *Pvu*II. A correct clone was visualised as the detachment of an ≈850 bp fragment, whereas an empty plasmid yielded a 602 bp fragment.

Destination vectors that were created by the LR reaction between the Entry clone plasmids (pIPentry3, pIHentryl and pEntryCIN1) and the Destination vector pBVboostFG were similarly carried out following the manufacturer's instructions. Mini preps were processed from the clones of the colonies resulting from the LR-reactions and verified by *Pvu*II digestions. The correct expression clones pDIP1-5 and pDIH1-5 were characterised by the appearance of 5 bands of the approximate sizes 4800, 2600, 800, 542 and 144 bp. The *Pvu*II digested intact plasmid pBVBoostFG was run next to the Destination plasmid digestions as a control (resulting in four bands of the sizes 4973, 2684, 1433 and 144 bps). The correct Expression plasmids containing cIN could be identified by the detachment of about 800 bps fragment upon digesting with *Pvu*II (Figure 5.1.5-IV). Clones giving the expected patterns were named pDIP, pDIH and pDcIN, the initial D standing for Destination plasmid.

Gene constructs in the final Expression vectors (clones pDIP2, pDIH1 and pDcIN1) were verified by sequencing with the primers listed in table 4.1.5-I. Sequencing results revealed the clones pDIP2, pDIH1 and pDcIN1 to be correct. These plasmid preparations were used for bacterial protein production (4.6)

### 5.2 Preparing the integration target plasmid (pPPOsite)

To prepare a plasmid that contains the cleavage site for I-PpoI, a double-stranded oligonucleotide containing the I-*Ppo*I recognition sequence was inserted into the *EcoR*V site of pBluescript. The preparation of the oligonucleotide linker (I-Ppo-oligo) comprising the 15 bp recognition sequence, as well as other steps in creating the plasmid pPPOsite, are described in section 4.3.

The pPPOsite-plasmid mini preps were verified by restriction analysis using the enzyme I*-Ppo*I (Promega). Clones bearing the I-Ppo-linker inserted became linearised upon digestion. One clone (pPPOsite#6) was further verified by sequencing the plasmid with the primers T7 and T3. Sequencing revealed that the I-Ppo-linker was incorporated as two copies in the *Eco*RV digested pBluescript. Sequence of the linker was correct.

### 5.3 Creating a substrate for integration (pB2LTR+EGFP)

Requirements for the HIV-1 integrase substrate are; that it should be blunt-ended, double-stranded DNA, flanked with at least 15 base pairs of each of the viral 5' and 3' long terminal repeat ends (LTRs) (Brown, 1997). The terminal 20 base pairs of the LTRs contain the crucial sequences needed for integration by HIV-1 IN, most importantly the dinucleotides 3'CA two bases away from each LTR's end (Brin & Leis, 2002a)

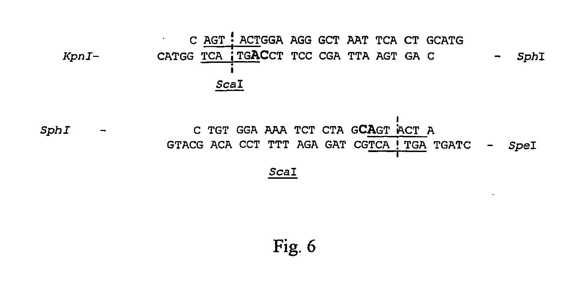

The LTR ends were designed to be easily clonable to pBluescript and later to allow the insertion of a marker gene cassette between the LTRs (see Figure 6). The 5' LTR was designed to fit to *Kpn*I-digested pBluescript from its 5' end and to the other LTR from its 3' end. The 3' LTR was designed to be ligated to pBluescript from its 3' end's *Spe*I site and to the 5' LTR from its *Sph*I site. In addition, extra endonuclease cleaving sites were introduced in both the LTR-sequences: *Sca*I sites partly embedded in LTR sequences would allow release of the LTR flanked blunt-ended EGFP cassette bearing the crucial CA dinucleotides in both its 3' ends, two nucleotides away from the LTR termini.

In Fig. 5, the upper sequence presents the 5' LTR and the lower corresponds to the 3' LTR The restriction site of *Sca*I is underlined and the CA-dinucleotides in each 3' end are bolded.

The preparation of the 5' and 3' LTRs, as well as their ligation into the *Kpn*I and *Spe*I digested pBluescript is described in section 4.4. Mini preps were processed from light blue colonies that most likely had the short LTR sequences (that only partially hindered the read-through of the α-fragment gene) inserted in the MCS. Plasmid preparations (pB2LTR #1-3) were verified by restriction analysis using *Sca*I (MBI). Insertion of the two LTRs created two restriction sites for *Sca*I in addition to the site already residing in pBluescript. A correct clone having both the LTRs was thus identified by the detachment of an 1160 bps long fragment from pBluescript (the sequence between the LTRs could not be detected due to its shortness). Before inserting the marker gene cassette between the LTRs, a pB2LTR-plasmid preparation (pB2LTR#1) was verified by sequencing. Sequencing results obtained with the primers T7 and T3 showed the plasmid to be as expected, i.e. having both the 5' and 3' LTRs correctly inserted.

The triple promoter containing EGFP marker gene cassette from pBVBoostFG-EGFP was cut using *Sph*I. The cassette was introduced into the pBluescript construct bearing the 5' and the 3' LTRs (pB2LTR#1) as described in 4.4. Insertion of the 2620 bp cassette could be verified by digesting mini preps with *Sca*I, which was designed to free the blunt-ended EGFP cassette. A correct clone was identified by the detachment of the EGFP-marker gene cassette (about 2620 bp) and two bands of about 1100 and 1800 bps resulting from the plasmid backbone. Restriction analysis showed 8/10 tested mini preps to be correct. Sequence of the EGFP cassette was assumed to be correct because it had not been modified after its sequence verification in pBVBoostFG. Orientation of the cassette in respect to the LTRs was not crucial because the cassette itself carried the promoter elements needed for the expression of EGFP.

### 5.4 Bacterial protein production and analysis

The fusion genes IN-I-PpoI and IN-H98A were created in order to study the functionality of the novel fusion proteins they encode, and to compare it with the activity of the native HIV-1 integrase (clone HXB2, cIN in this study). The genes were transferred into Destination vectors (pBVBoostFG) to be able to produce the proteins. Protein production experiments were carried out in bacterial cells using the *E.coli* strains BL21 (DE3) (Stratagene) and BL21-AI^{™} One Shot^{®} (Invitrogen) as expression hosts.

### 5.4.1 Sample harvesting and preparation for SDS-PAGE

The bacteria were transformed with the expression vectors pDIP2, pDIH1 and pDcIN1 as described in 4.1.7. As a positive control for expression studies, also the plasmid pINSD.His (Engelman and Craigie 1992, Craigie et al 1995) was transformed into the expression host bacteria. Bacterial colonies were grown in a shaking incubator until the cell density reached the OD₆₀₀ value of 0,6 - 1. At this stage, a part of the culture was inoculated in fresh LB_{g} media to compose the actual expression culture. Glycerol stocks were created from the remaining initial cultures of each clone. The expression culture was grown until the cell density reached the mid-log phase (OD₆₀₀ ≈0,4 ) in order to maximise the capacity of the bacteria to overexpress the wanted proteins. As a control for Western blot, untransformed bacterial samples were processed along the actual Expression (and positive control) samples.

Protein production in *E.coli* BL21 (DE3) cell culture was induced by adding IPTG (100mM) to a final concentration (c_{final}) of 1mM. Addition of IPTG induces the expression of the RNA polymerase T7 (T7RNAP) from the lacUV5 promoter. Expression of the T7 promoter can however be leaky, i.e. be "on" also in absence of the inducer in this cell. This can lead to uninduced expression of heterologous proteins, which may represent a disadvantage if the protein product is toxic. In effect, the gene product of pDIP2 was observed to hinder the growth of transformed bacteria on LB plates, as well as growth of bacterial cultures in LB media. Strict expression control was therefore a relevant issue in bacterial protein production.

BL21-AI^{™} One Shot^{®} cells were induced with the addition of IPTG (c_{final} 1-0,01 mM) and L-arabinose (c_{final} 0,2 %). L-arabinose induction was needed to induce expression of the T7RNAP from the *ara*BAD promoter (P_{BAD}) in BL21-AI (Lee 1980, Lee et al., 1987). Because T7 RNAP levels can be tightly regulated in BL21-AI, the strain was thought to be especially suitable to express possibly toxic genes. Various amounts of IPTG were screened to study the effects of different induction levels on the stability of the overexpressed proteins. Also different growing conditions (lower temperature, addition of glucose) were tested for the same purposes.

Both the strains BL21 (DE3) and BL21-AI are deficient of the proteases *lon* and OmpT (outer membrane protease) which cause degradation of expressed heterologous proteins in bacterial cells (Grodberg & Dunn, 1988; Studier & Moffat, 1986). Lack of these proteases thus reduces possibility of protein degradation in heterologous protein expression studies.

Time points of 1h, 2h, 3h and 4h (or some of them) were taken during expression experiments with both strains to define an ideal time for heterologous protein expression and stability. Samples were also taken from the cultures before induction (0h, UI) to assess the level of basal protein expression. All samples were harvested and prepared as described in section 4.7. Sample boiling immediately after cell pelleting was important in order to lyse the cells and inactivate the host's intracellular proteases. Part of the samples obtained from BL21 (DE3) transformants were frozen right after sample preparation (cell pelleting and addition of SDS-PAGE sample buffer) and boiled only before loading on the SDS-PAGE gel. Protein production was analysed by SDS-PAGE (4.7.1) and Western Blotting (4.7.2).

### 5.4.2 SDS-PAGE and Western Blotting

Denaturing SDS-PAGE was performed as described in 4.7.1. The proteins were blotted onto nitrocellulose membranes during Western blot and stained as described in 4.7.2. Staining with the poly-histidine recognising antibody revealed the sizes of fragments bearing the N-terminal 6XHis fusion, whereas staining with the HIV-1 integrase specific antibody revealed all fragments bearing the N-terminal region (amino acids 23-34) of the IN protein. An I-*Ppo*I specific antibody was not available for use, but the size difference of the proteins derived from pDIP2 and pDIH1 expression cultures compared to pDcIN derived proteins revealed the presence of correct fusion proteins. The protein product expressed from pDcIN was expected to be 32 kDa (as the monomeric wt HIV-1 integrase) and those of the IN-H98A (pDIH) and IN-I-PpoI (pDIP) fusions 50 kDa (32 kDa + I-PpoI or H98A 18 kDa per monomer). A positive control for the immunoblot staining was prepared from the plasmid pINSD.His in the same way that expression samples from pDcIN1, pDIP2 and pDIH1 were processed. This His-tagged control reacted with both antibodies used in this study and revealed the size of the cloned integrase and the presence of the poly-histidine tag in the protein's N-terminus.

Immunoblots derived from the expression samples in BL21 (DE3) showed the presence of expected protein bands resulting from specific antibody interactions in the staining procedure. In addition to the correct sized gene products of pDIH, pDIP and pDcIN, numerous smaller bands were present in the blots. Proteins were thought to be subjected to degradation at some point of the expression pathway, possibly due to the strong basal expression from the T7 promoter and the consequential large quantity of the heterologous proteins in the cells. Expression of the heterologous proteins occurred already at the uninduced state of the BL21 (DE3) culture which was thought to have contributed to protein instability.

Marked inhibition of bacterial growth was observed in cultured *E. coli* BL21 (DE3) cells when transformed with the expression plasmid pDIP2. Firstly, it was difficult to obtain any colonies when the bacteria were transformed with pDIP2. Secondly, both the initial culture as well as the expression culture of the pDIP2 transformed BL21 (DE3) cells grew very slowly if at all. Finally, the cells that could be extracted from the induced pDIP2 transformed BL21 (DE3) cultures had very little amounts of the expressed IN-1-*Ppo*I proteins.

To test whether the expression products of pDIP2, pDIH1 and pDcIN1 would be more stable when expressed in a strain more suitable for expression of toxic proteins, the experiments were repeated using the *E.coli* strain BL21-AI (Figures 5.4.2-II to 5.4.2-V). Immunoblotting results showed lower levels of basal expression in BL21-AI than in BL21 (DE3), but no clear enhancement in protein stability could be observed. Different levels of IPTG induction did not change the expression levels from DcIN1 and pDIH1, nor did they affect protein stability.

Expression of the IN-I-PpoI protein from pDIP2 was hindered, as already observed with pDIP2 infected BL21 (DE3) cells. The protein product was thus thought to be toxic for the expression hosts used in this study. Addition of glucose (0,1 %) to the LB plates and the growth media was tested with pDIP2, because it may prevent problems associated with the basal level expression from a toxic gene in BL21-AI cells [Manual for BL21-AI cells (Invitrogen) and references therein]. Glucose addition was found to have strong suppressive effects on the expression levels of the protein, but no significant improvement was observed on protein degradation.

### REFERENCES

Allen et al (1992) J Bacteriol. 174:6938-6947.
Argast et al (1998). J. Mol. Biol. 280:345-353.
Arnheim et al (1992). Annu. Rev. Biochem. 61: 131-156.
Belfort & Perlman (1995) J. Biol. Chem. 270: 30207-30240.
Belfort. & Roberts (1997). Nucleic Acids Res. 25: 3379-3388.
Bischerour et al (2003).J Virol. 77:135-141.
Blake et al (1984). Anal.Biochem. 136: 175-179.
Bouyac-Bertoia et al. (2001). Mol Cell. 7:1025-1035.
Brin & Leis (2002a) J. Biol. Chem. 277:10938-10948.
Brin & Leis (2002b) J. Biol. Chem. 277:18357-18364.
Brin et al (2000) J Biol Chem 275:39287-39295.
Brown (1997) Integration. In: Coffin, J. M., Hughes, S. H. & Varmus, H. E (Eds.) Retroviruses. Cold Spring Harbor Laboratory Press, Cold Spring Harbor, N.Y.
Brown et al (1987) Cell 3: 347-356.
Buchschacher & Wong-Staal (2000). Blood 95:2499-2504
Bukrinsky & Haffar,.(1999) Front. Biosci. 4:772-781.
Burns et al (1993) Proc. Natl. Acad. Sci. USA 90: 8033-8037.
Bushman & Craigie, (1991). Proc. Natl. Acad. Sci. USA 88:1339-1343.
Bushman et al (1990). Science 4976:1555-1558.
Bushman & Miller (1997). J. Virol. 71: 458-464.
Bushman (1994) Proc. Natl. Acad. Sci. USA 91: 9233-9237.
Bushman (1995) Science: 267: 1443-1444.
Carteau et al (1999). J. Virol 73:6670-6679.
Cavazzana-Calvo et al (2000) Science 288: 669-72.
Chen & Engelman, (1998) T Proc Natl Acad Sci USA. 95:15270-15274
Chevalier & Stoddard (2001). Nucleic Acids Res., 29: 3757-3774.
Chow (1997) Methods. 12:306-317.
Cooper (2000) In: The cell: a molecular approach, 2nd ed. Sinauer Associates, Inc Sunderland (MA)
Costantini et al (1999) Hum Gene Ther 10:2481-2494.
Craigie (2001) The Journal of Biological Chemistry 276:23213-23216.
Craigie et al (1990) Cell 62:829-837
Craigie et al (1995) In: HIV Volume 2: A Practical Approach. Ed J. Kam, Oxford Univ Press, p. 53.
Dalgaard et al (1993). Proc. Natl. Acad. Sci. USA 90:5414-5417.
Depienne et al (2001) J Biol Chem. 276:18102-18107.
Dong et al (1996) s Hum Gene Ther 7:2101-2112
Dyer & Herrling (2000). Molecular Therapy 1:213-224.
Dykxhoorn et al (2003) Nat Rev Mol Cell Biol. 4:457-467.
Ellison & Vogt (1993) I Mo/. Cell. Biol. 13:7531-7539.
Emi et al (1991). J Virol. 65:1202-1207.
Engelman & Craigie (1992) J Virol 66:6361-6369.
Engelman & Craigie (1995) J. Virol. 69:5908-5911.
Engelman et al (1991). Cell 67: 1211-1221.
Famet & Bushman (1997) Cell 88: 483-492.
Famet et al (1990) Proc. Natl. Acac. Sci. USA 87:4164-4168.
Flick et al. (1998).. Nature 394: 96-101.
Flick et al(1997) Protein Sci. 6: 2677-2680.
Frankel & Young (1998) Annu. Rev. Biochem. 67:1-25.
Freed & Martin (2001) HIVs and their replication. In: Knipe and Howley (Eds.) Fields Virology 4th edn. pp 1971-2401. Philadelphia: Lippincott Williams & Wilkins.
Galburt et al (2000). J. Mol. Biol. 300: 877-887.
Gallay et al (1997) Proc Natl Acad Sci USA. 94:9825-9830.
Georgiou (1996) Expression of proteins in bacteria. In: Protein Engineering: Principles and Practice pp. 101-127.Edited by Jeffrey L. Cleland and Charles S. Craik. Viley-Liss, Inc.
Gierse et al (1993) Protein Expr Purif.4: 358-366.
Gimble (2001) Nucleic Acids Research 29:4215-4223.
Gimble & Thorner (1992) Nature 357:301-306
Goff. (2001) Retroviridae: The retroviruses and their replication. In: Knipe and Howley (Eds.) Fields Virology 4th edn. pp 1871-1939.Philadelphia: Lippincott Williams & Wilkins.
Goulaouic& Chow (1996). LexA protein. J. Virol. 70: 37-46.
Gould & Favorov (2003)..Gene Therapy 10:912-927
Graham & Dickson (2002) Biochim Biophys Acta. 1587:1-6.
Grodberg & Dunn (1988) J Bacteriol. 170:1245-1253.
Guo et al (1997) The EMBO Journal 16: 6835-6848.
Haffar et al (2000) J Mol Biol. 299:3 59-368.
Hindmarsh et al. (1999) J. Virol. 73: 2994-3003
Holmes-Son & Chow (2000) J. Virol 24:11548-11556.
Holmes-Son & Chow (2002) Mol. Therapy 5:360-370
Hong et al (1993) J Virol 67:1127-1131
Hu & Pathak (2000) Pharmacol. Rev. 52: 493-511.
Hunter (1997) Viral Entry and Receptors. (Chapter 3) In: Coffin, J. M., Hughes, S. H. & Varmus, H. E (Eds.) Retroviruses. Cold Spring Harbor Laboratory Press, Cold Spring Harbor,N.Y
Hüser & Hofmann (2003) Am J Pharmacogenomics 3: 53-63
Johansen et al (1993). Nuceicl. Acids Res. 21:4405-4411.
Jurica & Stoddard (1999). CMLS, Cell. Mol. Life Sci. 55:1304-1326.
Kalpana et al. (1994) Science; 266:2002-2006
Katz et al (1990) Cell 63:87-95
Katz. & Skalka (1994). Annu. Rev. Biochem. 63:133-173.
Kay et al. (2001) Nat Med. Jan;7:33-40.
Kearns et al (1996). Gene Ther. 3:748-755.
Kondo et al (1996). J Virol. 70:159-64.
Kootstra, & Verma (2003) Annu. Rev. Pharmacol. Toxicol. 43:413-439.
Kost & Condreay (2002) Trends Biotechnol 20:173-180.
Landy (1989) Annu Rev Biochem 58:913-949.
Lee (1980) Molecular Aspects of ara Regulation. In The Operon, J. H. Miller and W. S. Reznikoff, eds. Cold Spring Harbor, N.Y. :Cold Spring Harbor Laboratory, pp. 389-410.
Lee et al (1987). Proc. Natl. Acad. Sci. USA 84: 8814-8818.
Lee & Coffin (1990) J. Virol. 12:5958-65.
Leh et al (2000) Biochemistry 39:9285-9294.
Lewin (2000) Genes VII, Oxford University Press Inc. New York
Li et al (2002) Murine leukemia induced by retroviral gene marking.
Lin & Vogt (1998). Mol. Cell. Biol. 18: 5809-5817.
Lin & Vogt (2000) Nucleic Acids Res. 28:1428-1438.
Loizos et al (1994) Proc Natl Acad Sci U S A. 91: 11983-11987
Lowery et al (1992) Promega Notes Magazine 38:8
Luckow et al (1993) J Virol. 67:4566-4579.
Mannino et al (1999) Biochemistry 38: 16178-16186.
Marshall (2003) Science 299:320
Miao et al (1998) Nat Genet. 19:13-15.
Michelsen (1995) Anal Biochem. 225:172-174.
Miller (1997) Development and Applications of Retroviral Vectors (Chapter 9) In: Coffin, J. M., Hughes, S. H. & Varmus, H. E (Eds.) Retroviruses. Cold Spring Harbor Laboratory Press, Cold Spring Harbor, N.Y.
Miller et al. (2002). Nature Genet. 30: 147-148.
Miller et al. (1990) Mol. Cell Biol. 10:4239-4242.
Miller et al (1995). Curr. Biol. 5: 1047-1056.
Monnat et al (1999) Biochem. Biophys. Res. Communications 255: 88-93.
Murphy et al (2002) Hum. Gene Ther. 13:745-760.
Muscarella & Vogt (1993) Mol. Cell. Biol. 13:1023-1033.
Muscarella & Vogt (1989). Cell 56: 443-454.
Muscarella et al (1990).. Mol. Cell. Biol. 10: 3386-3396.
Nakai et al (2003) Nat Genet. 34:297-302.
Nakai et al (2001). J. Virol. 75:6969-6976.
Olivares et al (2002) Nature Biotechnology 20: 1124-1128.
Ortiz-Urda et al(2002) Nature Medicine 8: 1166-1170.
Palombo et al (1998) J Virol. 72:5025-34.
Pannell & Ellis (2001) Rev. Med. Virol. 11:205-217.
Pavletich & Pabo. (1991). Science 252: 809.
Pfeifer & Verma (2001) Virus vectors and their applications. In: Knipe and Howley (Eds.) Fields Virology 4th edn. Philadelphia: Lippincott Williams & Wilkins.
Pruss et al (1994) Proc. Natl. Acad. Sci. USA 91:5913-5917.
Recchia et al (1999) Proc Natl Acad Sci USA 96:2615-2620.
Reicin et al. (1995). J. Virol. 69:5904-5907.
Rice et al. (1996). Curr Opin Struct Biol. 6: 76-83
Robinett et al (1997) Mol. Cell. Biol. 17: 2866-2875
Roe, et al. (1993) EMBO J. 12: 2099-2108.
Ruoff et al (1992) m.Nucleic Acids Res. 20:5899-5906
Sambrook, Russel. (2001). Molecular cloning. A laboratory manual third edition. Cold Spring Harbor Laboratory Press. Cold Spring Harbor, NY.
Satoh et al (2000). J Virol 74:10631-10638
Schröder et al. (2002) Cell 110:521-529
Shagen et al. (2000). Gene Therapy 7: 271-272.
Sherman et al. (2001) J Virol 75:1522-1532
Sherman et al (1992) J. Virol. 66:5393-601.
Sherman & Fyfe (1990) Proc. Natl. Acad. Sci. USA 87:5119-5123.
Sinha et al (2002) J. Virol. 76:3105-3113.
Sioud & Drilca (1991) Proc Natl Acad Sci USA 88:7303-7307
Studier & Moffatt (1986) J Mol Biol. 189:113-130
Thomas et al (2003) NatRev Genet. 4:346-358
? et al (2001).. Mol. Cell. Biol. 21: 3926-3934.
Turner & Summers. (1999) J Mol Biol. 285:1-32.
Ueno et al (2000) Biochem Biophys Res Commun. 273:473-478
Verma, & Somia (1997). Nature 389: 239-242.
Vigdal et al (2002) J. Mol. Biol. 323:441-452.
Vink et al (1990) J. Virol. 64:5626-5627
Vogt (1997) Retroviral Virions and Genomes (Chapter 2) In: Coffin, J. M., Hughes, S. H. & Varmus, H. E (Eds.) Retroviruses. Cold Spring Harbor Laboratory Press, Cold Spring Harbor, N.Y.
Weitzman et al. (1994) Proc. Natl Acad Sci USA 91:5808-5812
Wiesendanger et al (1994) Nucleic Acids Res. 22:5038-46.
Wittmayer et al (1996) Biochemistry 35: 1076-1083.
Wittmayer et al. (1998) Gene 206: 11-21.
Yant et al. (2002). Nature Biotechnol. 20:999-1005.
Yant et al (2000). Nature Genetics 25:35-41.
Zheng et al (1996) Proc. Natl. Acad. Sci. USA 93:13659-13664.
Zimmerly et al. (1999) J. Mol Biol. 289: 473-490.
Lu et al (1995) J Virol. 69:6873-9.
Wu et al (1995) J. Virol. 69:3389-3398
Wu et al (1996). J. Virol. 70:3378- 3384.
Wu et al (1996). Virology 219:307-313.
Wu et al (1997) EMBO J. 16:5113-5122.

The content of any document identified herein is incorporated herein by reference.

### APPENDIX I

**Table I-1 Primers used in insert-PCR (4.1.1.1). The sequences are presented in the 5' → 3' direction. RE-sites introduced in the primers are presented as underlined sequences and explained in the table. Start- and stop codons are bolded.**

| Primer name | Sequence | RE-site | Template& PCR product |
|---|---|---|---|
| F992 | CCTTAATTAAATGTTTTTAGATGGA ATAGAT | PacI | PLJS10, IN |
| 3'IN | GCTCTAGAATCCTCATCCTGTCTACT | XbaI | --"--, IN, cIN |
| 3'cIN | TATGGCCTCTCAGGCCATTATTAAT CCTCATCCTGTCTACT | SfiI | --"--, cIN |
| G7 | ATTCACCACTAGTGCTCCAAAAAA AAGCGC | SpeI | pCNPpo6:I-PpoI pCNPpo6h98a: H98A |
| F987 | TATGGCCTCTCAGGCCATTATTATA CCACAAAGTGACTGCC | SfiI | ----"----- |

**Table I-2 Primers used in GATEWAY-PCR (4.1.7.1). The sequences are presented in the 5' → 3' direction. Two stop codons in GW 3'Ppo- and GW 3'cIN primers, as well as a start codon in the GW 5'IN HT, are marked in bolded. The six histidine encoding codons in GW 5'IN HT are underlined.**

| Primer name | Sequence |
|---|---|
| G238 (GW 3'Ppo) | GGGGACCACTTTGTACAAGAAAGCTGGGTTATGGCC TCTCAGGCCATTATTATACCACAAAGTGACTGCC |
| G402 (GW 3' cIN) | GGGGACCACTTTGTACAAGAAAGCTGGGTATTATTA ATCCTCATCCTGTCTACT |
| G445 (GW 5'IN HT) | GGGACAAGTTTGTACAAAAAAGCAGGCTATG CATCACCATCACCATCACCTGGTGCCGCGCGGCAGC TTTTTAGATGGAATAGAT |

**Table I-3: Primers used in sequencing. The sequences are presented in the 5' → 3' direction.**

| Primer name | Sequence |
|---|---|
| G448 | GGGGAAAGAATAGTAGAC |
| G449 | GCCACACAATCATCACCTGCC |
| T3 | ATT AAC CCT CAC TAA AGG G |
| T7 | AAT ACG ACT CAC TAT AGG G |
| G502 | CAATCAAAGGAGATATACCACG |
| G550 | TCGACCTGCAGGCGCGCCGA |

**Table I-4: Oligodeoxyribonucleotides used in creation of the LTRs for pB2LTR and in construction of the I-PpoI site inserted in pPPOsite.**

| Oligo name | Sequence | Description |
|---|---|---|
| G515 | CTCTCTTAAGGTAGC | I-PpoI upper |
| G517 | GCTACCTTAAGAGAG | I-PpoIlower |
| G569 | CTAGTAGTACTGCTAGAGATTTTCCACAGCATG | 3' LTR lower |
| G570 | CTGTGGAAAATCTCTAGCAGTACTA | 3'LTR upper |
| G604 | CAGTGAATTAGCCCTTCCAGTACTGGTAC | 5'LTR lower |
| G605 | CAGTACTGGAAGGGCTAATTCACTGCATG | 5'LTR upper |
| G448 | GGGGAAAGAATAGTAGAC | 5'newSeq4IP |
| G449 | GCCACACAATCATCACCTGCC | 3'NewSeq4IP |

### APPENDIX II

**Table II-1 PCR programs used in the study**

| DS2006 | DPESÄKE | GW2701 | GW-cIN |
|---|---|---|---|
| (insert PCR) | (Colony-PCR) | GATEWAY-PCR | |
| 1. 94°C 1 min. | 1. 95°C 5 min | 1. 96°C 1min 30 sec | 1. 96°C 1min 30 |
| 2. 94°C 30 sec. | 2.95C 1min | 2. 94°C 1min | sec |
| 3. 50°C 30 sec. | 3. 51°C 30 sec. | 3. 52,5°C 30 sec | 2. 94°C 1min |
| 4. 72°C 1 min | 4. 72°C 1 min 35 s | 4.72°C 2 min | 3. 52,5°C 30 sec |
| 5. 25 x cycles 2-4 | 5. 25 x cycles 2-4 | 5. 25 x cycles 2-4 | 4.72°C 1 min |
| 6.72°C 5 min | 6. 72°C 6 min | 6.72°C 5min | 5. 25 x cycles 2-4 |
| 7. 4°C ∞ | 7. 4°C ∞ | 7. 4°C ∞ | 6. 72°C 5min |
| | | | 7. 4°C ∞ |

### APPENDIX III

1) GeneRuler™ 100bp DNA Ladder (MBI)
2) GeneRuler™ 1 kb DNA Ladder (MBI)
3) GeneRuler™ DNA Ladder Mix (MBI)
4) 1 Kb DNA Ladder (NEB)

### APPENDIX IV:

### Buffers and reagents

| | |
|---|---|
| Ammonium Persulfate 10% (APS) | 0,1 g APS, dissolve in 1 ml H₂O |
| | Prepare just before use or store at -20°C. |
| APA-buffer | 0,1 M NaHCO₃ |
| | 1 mM MgCl₂.6·H₂O |
| | Adjust pH with NaOH to 9.8 |
| Kodak (Transfer buffer) | 100 ml 10x Kodak stock solution |
| | 200 ml methanol |
| | 700 ml H₂O |
| | 1L |
| Kodak (Transfer buffer) 10 x stock | 30,3 g Tris base |
| solution | 30,3 g Glycine |
| | Add H₂O to 1000 ml |
| LB-solution | 10 g Bacto-tryptone |
| | 5 g Bacto-yeast extract |
| | 10 g NaCl |
| | Adjust pH to 7.5 with 1M NaOH, fill to |
| | 100 ml with H₂O |
| PBS-buffer | 276 mM NaCl |
| | 16 mM Na₂HPO₄ |
| | 10,7 mM KCl |
| | 2,9 mM KH₂PO₄ |
| Ponceau protein staining solution | 0,2% Ponceau S |
| | 3% TCA |
| Running buffer 5 X, pH 8.3 | 15 g Tris base |
| | 72 g Glycine |
| | 5 g SDS (Sodium dodecyl sulphate) |
| | Add H₂O to 700 ml. Adjust pH. Add water |
| | to a final volume of 1000 ml. Store at |
| | +4°C. |
| SDS-PAGE Sample buffer (2x) | 2,5 ml 0,5M Tris-HCl (pH 6.8) |
| | 4,0 ml 10% SDS |
| | 2,0 ml glycerol |
| | 0,2 ml 0,2% bromophenole blue |
| | 1,0 ml β-mercaptoethanol |
| | 0,3 ml H₂O |
| SOC | 2 g Bacto-tryptone |
| | 0,5 g Yeast-extract |
| | 1 ml 1M NaCl |
| | 0,25 ml 1M KCl |
| | 1 ml 2 M Mg-stock solution |
| | 1 ml 2 M glucose |
| | Adjust volume to 100 ml with H₂O |
| TAE- buffer | 0,04 M Tris-acetate |
| | 1 mM EDTA |
| TBS 0,5M | 29,22 g NaCl |
| | 3,15 g Tris-HCl |
| | Add H₂O to 1000 ml |
| TBS- buffer | 20 mM Tris-HCl (pH 7.5) |
| | 0,5 M NaCl |
| TBS-Tween-buffer | 1 x TBS buffer |
| | 0,2% (v/v) Tween20 |
| TE-buffer | 10 mM Tris-HCl pH 8 |
| | 1 mM EDTA |
| Tris-HCl 0,5 M, pH 6.8 | 6 g Tris base; dissolve in 60 ml H₂O |
| | Adjust pH to 6.8, add H₂O to 100 ml |
| | Store at +4°C. |
| Tris-HCl 1,5 M, pH 8.8 | 27,23 g Tris base; dissolve in 80 ml H₂O |
| | Adjust pH to 8.8, add H₂O to 150 ml |
| | Store at+4°C |

### Preparation of SDS-PAGE gels:

| | |
|---|---|
| **10% Running Gel (thick comb)** | **4% Stacking gel (thick comb)** |
| 6,023 ml Distilled H₂O | 6,10 ml Distilled H₂O |
| 3,75 ml 1,5 M Tris-HCl pH 8.8 | 2,50 ml 0,5 M Tris-HCl pH 6.8 |
| 150 µl 10% SDS-stock | 100 µl 10% SDS-stock |
| 5,00 ml 30% acrylamide/bis-solution | 1,30 ml 30% acrylamide/bis-solution |
| (BioRad) | (BioRad) |
| 75 µl 10% APS | 50 µl 10% APS |
| 7,5 µl TEMED | 10 µl TEMED |

## Claims

1. A fusion protein which comprises an endonuclease fused to an integrase, wherein the endonuclease is specific to a site in an abundant rDNA locus and is fused to an integrase that mediates the introduction of a transgene comprising retrovirus-like nucleic acid.

2. A protein according to claim 1, wherein the endonuclease is I-Ppol.

3. A protein according to claim 1, wherein the endonuclease is Crel.

4. A protein according to claim 1, wherein the endonuclease is H98A.

5. A protein according to any preceding claim, wherein the integrase is a lentivirus integrase.

6. A protein according to claim 5, wherein the integrase is a HIV-1 integrase.

7. A protein according to claim 1, which is of HIV-1 integrase and I-Ppol.

8. A fusion protein according to any preceding claim, for use in therapy.

9. A method of targeting integration of a transgene comprising retrovirus-like nucleic acid into a eukaryotic genome *in vitro,* in which the genome is targeted by endonuclease that binds nucleic acid and the transgene is introduced at the binding site, wherein the endonuclease is as defined in claim 1, and is fused to an integrase as defined in claim 1.

10. A method according to claim 9, wherein the genome is human.

## Patentansprüche

1. Fusionsprotein, umfassend eine Endonuclease fusioniert mit einer Integrase, wobei die Endonuclease spezifisch ist für eine Stelle in einem abundanten rDNA-Locus und fusioniert ist mit einer Integrase, welche die Einführung eines Transgens vermittelt, das eine Retrovirus-ähnliche Nucleinsäure umfasst.

2. Protein nach Anspruch 1, wobei die Endonuclease I-Ppol ist.

3. Protein nach Anspruch 1, wobei die Endonuclease Crel ist.

4. Protein nach Anspruch 1, wobei die Endonuclease H98A ist.

5. Protein nach irgendeinem vorangehenden Anspruch, wobei die Integrase eine Lentivirus-Integrase ist.

6. Protein nach Anspruch 5, wobei die Integrase eine HIV-1-Integrase ist.

7. Protein nach Anspruch 1, welches von HIV-1-Integrase und I-Ppol ist.

8. Fusionsprotein nach irgendeinem vorangehenden Anspruch zur Verwendung in der Therapie.

9. Verfahren zum Targetieren der Integration eines Transgens, das Retrovirus-ähnliche Nucleinsäure umfasst, in vitro in ein eukaryotisches Genom, bei dem das Genom durch Endonuclease targetiert wird, welche Nucleinsäure bindet, und das Transgen an der Bindungsstelle eingeführt wird, wobei die Endonuclease wie in Anspruch 1 definiert ist und mit einer Integrase fusioniert ist, die wie in Anspruch 1 definiert ist.

10. Verfahren nach Anspruch 9, wobei das Genom human ist.

## Revendications

1. Protéine de fusion qui comprend une endonucléase fusionnée à une intégrase, dans laquelle l'endonucléase est spécifique pour un site dans un locus de rADN abondant, et est fusionnée à une intégrase qui induit l'introduction d'un transgène comprenant une acide nucléique de type rétroviral.

2. Protéine selon la revendication 1, dans laquelle l'endonucléase est I-Ppol.

3. Protéine selon la revendication 1, dans laquelle l'endonucléase est Crel.

4. Protéine selon la revendication 1, dans laquelle l'endonucléase est H98A.

5. Protéine selon l'une quelconque des revendications précédentes, dans laquelle l'intégrase est une intégrase lentivirale.

6. Protéine selon l'une quelconque des revendications précédentes, dans laquelle l'intégrase est une intégrase du VIH-1.

7. Protéine selon la revendication 1, qui est formée d'intégrase du VIH-1 et de I-Ppol.

8. Protéine de fusion selon l'une quelconque des revendications précédentes, à utiliser en thérapie.

9. Procédé pour cibler une intégration d'un transgène comprenant un acide nucléique de type rétroviral dans un génome d'eucaryote *in vitro,* dans lequel le génome est ciblé par une endonucléase qui lie l'acide nucléique, et le transgène est introduit au site de liaison, dans lequel l'endonucléase est telle que définie à la revendication 1, et est fusionnée avec une intégrase telle que définie à la revendication 1.

10. Procédé selon la revendication 9, dans lequel le génome est humain.
